# EUROPEAN PATENT APPLICATION

(11) **EP 4 831 184 A1**
(43) Date of publication of application: **28.10.2026**
(21) Application number: 25736346.5
(22) Date of filing: 03.01.2025
(51) Int. Cl.: C07D 519/00, C07D 487/04, A61K 31/519

(54) **SALT OF NITROGEN HETEROARYL COMPOUND, CRYSTAL FORM OF SAID SALT, PREPARATION METHOD THEREFOR, AND USE THEREOF**

(30) Priority: 05.01.2024 CN 202410016874
(71) Applicant: Shanghai Aolu Biomedical Technology Co., Ltd., Shanghai 201203 (CN)
(72) Inventor: WU, Guaili, Shanghai 201203 (CN); SHEN, Jiaqi, Shanghai 201203 (CN); MA, Shichao, Shanghai 201203 (CN)
(74) Representative: Isarpatent
(86) International application number: PCT/CN2025/070463
(87) International publication number: WO 2025/146142

(57) **Abstract**

Provided in the present invention are a salt of a nitrogen heteroaryl compound, a crystal form of said salt, a preparation method therefor, and use thereof. Provided in the present invention is a p-toluenesulfonate or hydrochloride of a compound represented by formula (I). The salt of the compound represented by formula (I) provided in the present invention has a higher AUC and Cₘₐₓ, a faster in vivo peak time, a higher exposure amount, and good pharmacokinetic properties.

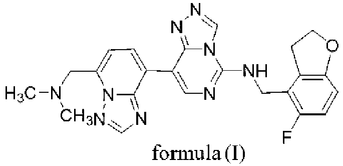

## Description

The present application claims priority to Chinese Patent Application No. 202410016874X filed on January 5, 2024. The contents of the above Chinese patent application are incorporated herein by reference in their entirety.

### TECHNICAL FIELD

The present disclosure relates to salts of a nitrogen heteroaryl compound, crystal forms of the salts, preparation methods therefor, and use thereof.

### BACKGROUND

EED (embryonic ectoderm development) and EZH2 (enhancer of zeste homolog 2) are two core proteins of the PRC2 complex (polycomb repressive complex 2). PRC2 inhibits the expression of related genes through methylation modification of lysine at position 27 of histone 3 (H3K27) in chromatin. The binding of EED to trimethylated H3K27Me3 not only exerts a strong allosteric enhancement effect on the enzymatic catalytic function of EZH2, but also localizes the PRC2 complex to the chromatin that requires modification. Dysfunction of PRC2, such as overexpression or gain-of-function mutations of EZH2, is associated with many tumor diseases in clinical practice, including lung cancer, breast cancer, rectal cancer, prostate cancer, bladder cancer, pancreatic cancer, sarcoma, lymphoma, etc. PRC2 is also associated with various cellular immune functions. For example, EZH2 participates in the regulation of lymphocyte activation and can cooperate with glycolysis to promote T cell responses against tumor cells. Therefore, the research and development of small-molecule inhibitors targeting PRC2 possesses significant and broad value in drug discovery.

Research and development efforts centered on PRC2 inhibitors mainly follow two strategies: the development of EZH2 inhibitors and the development of EED inhibitors. EZH2 inhibitors that have entered clinical trials to date include EPZ-6438 (Epizyme, phase II clinical trial), GSK2816126 (GSK, phase I clinical trial), CPI-1205 (Constellation, phase I clinical trial), etc. Although multiple EZH2 inhibitors have advanced into clinical trials, these inhibitors all contain a common 2-pyridone pharmacophore. Moreover, secondary mutations have already begun to emerge in clinical treatment with existing EZH2 inhibitors. EED inhibitors exert an allosteric inhibitory effect on the enzymatic function of EZH2, and can achieve biological functions that are equivalent or similar to those of EZH2 inhibitors. Furthermore, in one aspect, EED inhibitors can effectively overcome the problem of EZH2 drug resistance, and in another aspect, EED inhibitors can be used in combination with EZH2 inhibitors to achieve better synergistic effects. Currently, the only EED inhibitor that has entered clinical trials is MAK683 (Novartis, phase II clinical trial). Therefore, it is of great significance to develop novel solid forms of EED inhibitors and conduct drug developability studies thereon.

### SUMMARY

Disclosed in the present disclosure are salts of an azaaryl compound, crystal forms of the salts, preparation methods therefor, and use thereof. The salts of the azaaryl compound of the present disclosure have good pharmacokinetic properties, and the crystal forms thereof exhibit good solubility and physicochemical stability, showing promising prospects for drug development.

The present disclosure provides a p-toluenesulfonate of a compound represented by formula (I), 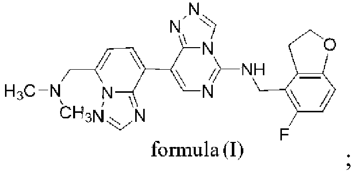 wherein the molar ratio of the compound represented by formula (I) to p-toluenesulfonic acid is 1:1.

The present disclosure provides a crystal form I of a p-toluenesulfonate of a compound represented by formula (I), wherein an X-ray powder diffraction (XRPD) pattern of the crystal form, measured using Cu-Kα radiation and expressed in terms of 2θ angles, has diffraction peaks at 6.63±0.20°, 11.91±0.20°, 13.24±0.20°, 14.03±0.20°, 15.71±0.20°, and 18.14±0.20°, 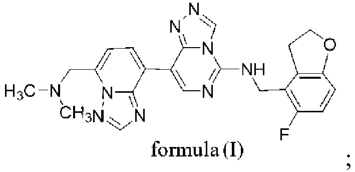 the molar ratio of the compound represented by formula (I) to p-toluenesulfonic acid is 1:1.

In a certain embodiment, the X-ray powder diffraction (XRPD) pattern of the crystal form I of the p-toluenesulfonate of the compound represented by formula (I), measured using Cu-Kα radiation and expressed in terms of 2θ angles, further has diffraction peaks at one or more of 17.24±0.20°, 21.92±0.20°, 22.54±0.20°, 24.79±0.20°, 25.06±0.20°, and 26.63±0.20°.

In a certain embodiment, the X-ray powder diffraction (XRPD) pattern of the crystal form I of the p-toluenesulfonate of the compound represented by formula (I), measured using Cu-Kα radiation and expressed in terms of 2θ angles, further has diffraction peaks at one or more of 10.87±0.20°, 14.29±0.20°, 17.70±0.20°, 18.99±0.20°, 21.63±0.20°, and 33.21±0.20°.

In a certain embodiment, the X-ray powder diffraction (XRPD) pattern of the crystal form I of the p-toluenesulfonate of the compound represented by formula (I), measured using Cu-Kα radiation and expressed in terms of 2θ angles, has diffraction peaks at 6.63±0.20°, 10.87±0.20°, 11.91±0.20°, 13.24±0.20°, 14.03±0.20°, 14.29±0.20°, 15.71±0.20°, 17.24±0.20°, 18.14±0.20°, 22.54±0.20°, 24.79±0.20°, 25.06±0.20°, and 26.63±0.20°.

In a certain embodiment, the X-ray powder diffraction (XRPD) pattern of the crystal form I of the p-toluenesulfonate of the compound represented by formula (I), measured using Cu-Kα radiation and expressed in terms of 2θ angles, exhibits diffraction peak positions as shown in the following table:

| No. | 2θ (°) | No. | 2θ (°) | No. | 2θ (°) | No. | 2θ (°) |
|---|---|---|---|---|---|---|---|
| 1 | 5.98 | 23 | 23.26 | 45 | 34.56 | 67 | 47.19 |
| 2 | 6.63 | 24 | 23.55 | 46 | 34.91 | 68 | 47.45 |
| 3 | 8.82 | 25 | 24.06 | 47 | 35.11 | 69 | 48.19 |
| 4 | 10.87 | 26 | 24.79 | 48 | 35.68 | 70 | 49.04 |
| 5 | 11.91 | 27 | 25.06 | 49 | 36.18 | 71 | 49.64 |
| 6 | 13.24 | 28 | 26.03 | 50 | 36.75 | 72 | 50.02 |
| 7 | 14.03 | 29 | 26.63 | 51 | 36.99 | 73 | 50.56 |
| 8 | 14.29 | 30 | 27.27 | 52 | 37.49 | 74 | 50.71 |
| 9 | 15.71 | 31 | 27.49 | 53 | 37.95 | 75 | 51.59 |
| 10 | 16.37 | 32 | 28.02 | 54 | 38.28 | 76 | 52.00 |
| 11 | 17.24 | 33 | 28.32 | 55 | 38.67 | 77 | 52.39 |
| 12 | 17.70 | 34 | 28.68 | 56 | 39.19 | 78 | 53.51 |
| 13 | 18.14 | 35 | 29.23 | 57 | 40.40 | 79 | 54.70 |
| 14 | 18.99 | 36 | 29.84 | 58 | 40.91 | 80 | 55.04 |
| 15 | 19.76 | 37 | 30.20 | 59 | 41.57 | 81 | 55.81 |
| 16 | 20.14 | 38 | 30.71 | 60 | 41.99 | 82 | 56.39 |
| 17 | 20.44 | 39 | 31.13 | 61 | 42.79 | 83 | 56.96 |
| 18 | 20.86 | 40 | 31.59 | 62 | 43.29 | 84 | 57.76 |
| 19 | 21.24 | 41 | 32.30 | 63 | 43.98 | 85 | 58.41 |
| 20 | 21.63 | 42 | 32.79 | 64 | 44.82 | 86 | 58.91 |
| 21 | 21.92 | 43 | 33.21 | 65 | 45.82 | 87 | 59.45 |
| 22 | 22.54 | 44 | 33.90 | 66 | 46.66 | | |

In a certain embodiment, the X-ray powder diffraction (XRPD) pattern of the crystal form I of the p-toluenesulfonate of the compound represented by formula (I), measured using Cu-Kα radiation and expressed in terms of 2θ angles, exhibits diffraction peak positions and relative intensities as shown in the following table:

| No. | 2θ (°) | Relative intensity (%) | No. | 2θ (°) | Relative intensity (%) |
|---|---|---|---|---|---|
| 1 | 5.98 | 0.3 | 45 | 34.56 | 0.5 |
| 2 | 6.63 | 93.0 | 46 | 34.91 | 0.3 |
| 3 | 8.82 | 0.2 | 47 | 35.11 | 0.2 |
| 4 | 10.87 | 3.7 | 48 | 35.68 | 0.5 |
| 5 | 11.91 | 26.2 | 49 | 36.18 | 0.6 |
| 6 | 13.24 | 100.0 | 50 | 36.75 | 0.3 |
| 7 | 14.03 | 11.5 | 51 | 36.99 | 0.3 |
| 8 | 14.29 | 3.6 | 52 | 37.49 | 0.4 |
| 9 | 15.71 | 12.9 | 53 | 37.95 | 0.1 |
| 10 | 16.37 | 0.2 | 54 | 38.28 | 0.1 |
| 11 | 17.24 | 6.7 | 55 | 38.67 | 0.5 |
| 12 | 17.70 | 2.5 | 56 | 39.19 | 0.1 |
| 13 | 18.14 | 12.8 | 57 | 40.40 | 2.1 |
| 14 | 18.99 | 2.1 | 58 | 40.91 | 0.5 |
| 15 | 19.76 | 0.1 | 59 | 41.57 | 0.3 |
| 16 | 20.14 | 0.2 | 60 | 41.99 | 1.5 |
| 17 | 20.44 | 0.2 | 61 | 42.79 | 0.1 |
| 18 | 20.86 | 1.0 | 62 | 43.29 | 0.3 |
| 19 | 21.24 | 1.9 | 63 | 43.98 | 0.5 |
| 20 | 21.63 | 3.2 | 64 | 44.82 | 0.6 |
| 21 | 21.92 | 5.8 | 65 | 45.82 | 0.1 |
| 22 | 22.54 | 5.4 | 66 | 46.66 | 0.0 |
| 23 | 23.26 | 0.3 | 67 | 47.19 | 1.6 |
| 24 | 23.55 | 0.7 | 68 | 47.45 | 0.3 |
| 25 | 24.06 | 1.4 | 69 | 48.19 | 0.4 |
| 26 | 24.79 | 5.3 | 70 | 49.04 | 0.6 |
| 27 | 25.06 | 6.3 | 71 | 49.64 | 0.2 |
| 28 | 26.03 | 1.8 | 72 | 50.02 | 0.3 |
| 29 | 26.63 | 8.8 | 73 | 50.56 | 0.2 |
| 30 | 27.27 | 0.5 | 74 | 50.71 | 0.2 |
| 31 | 27.49 | 0.9 | 75 | 51.59 | 0.2 |
| 32 | 28.02 | 1.1 | 76 | 52.00 | 0.1 |
| 33 | 28.32 | 0.9 | 77 | 52.39 | 0.1 |
| 34 | 28.68 | 0.3 | 78 | 53.51 | 0.1 |
| 35 | 29.23 | 0.1 | 79 | 54.70 | 0.2 |
| 36 | 29.84 | 0.3 | 80 | 55.04 | 0.4 |
| 37 | 30.20 | 0.4 | 81 | 55.81 | 0.1 |
| 38 | 30.71 | 1.1 | 82 | 56.39 | 0.1 |
| 39 | 31.13 | 0.3 | 83 | 56.96 | 0.1 |
| 40 | 31.59 | 1.0 | 84 | 57.76 | 0.2 |
| 41 | 32.30 | 1.1 | 85 | 58.41 | 0.1 |
| 42 | 32.79 | 0.4 | 86 | 58.91 | 0.0 |
| 43 | 33.21 | 3.2 | 87 | 59.45 | 0.1 |
| 44 | 33.90 | 0.8 | | | |

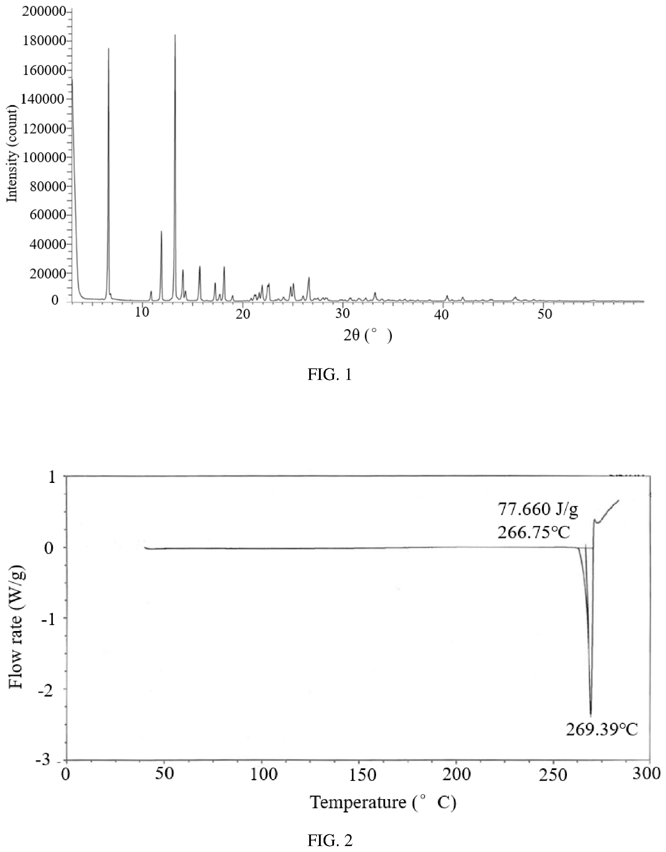

In a certain embodiment, the X-ray powder diffraction (XRPD) pattern of the crystal form I of the p-toluenesulfonate of the compound represented by formula (I), measured using Cu-Kα radiation and expressed in terms of 2θ angles, is substantially as shown in FIG. 1.

In a certain embodiment, a differential scanning calorimetry (DSC) curve of the crystal form I of the p-toluenesulfonate of the compound represented by formula (I) has an endothermic peak onset at 266.75±3 °C, and/or the differential scanning calorimetry (DSC) curve of the crystal form I of the p-toluenesulfonate of the compound represented by formula (I) has a peak temperature of 269.39±3 °C.

In a certain embodiment, the differential scanning calorimetry (DSC) curve of the crystal form I of the p-toluenesulfonate of the compound represented by formula (I) is substantially as shown in FIG. 2.

In a certain embodiment, a thermogravimetric analysis (TGA) curve of the crystal form I of the p-toluenesulfonate of the compound represented by formula (I) shows no weight loss below 100±3 °C (e.g., in a range of 25±3 °C to 100±3 °C, such as in a range of 50±3 °C to 100±3 °C).

In a certain embodiment, the thermogravimetric analysis (TGA) curve of the crystal form I of the p-toluenesulfonate shows that the crystal form undergoes decomposition above 250±3 °C.

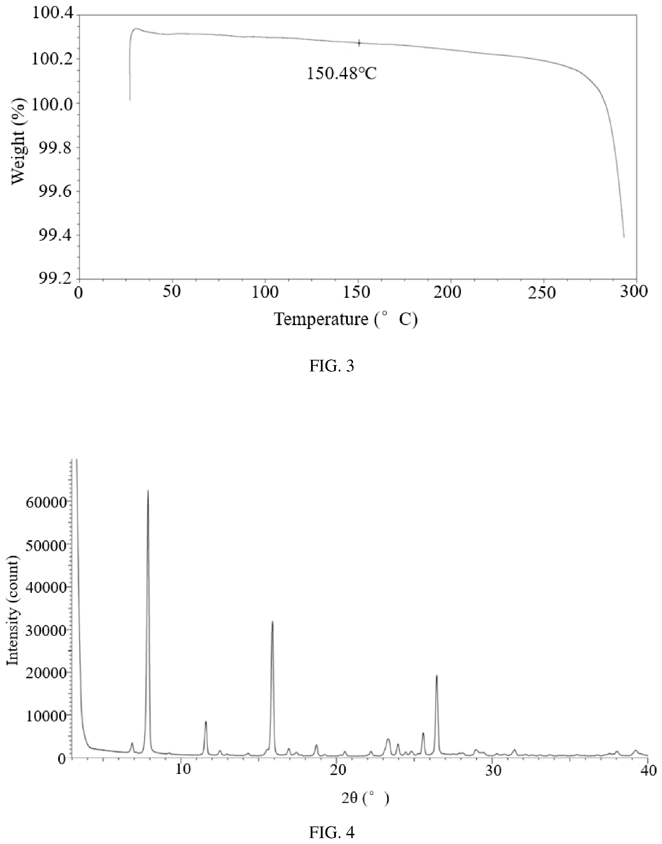

In a certain embodiment, the thermogravimetric analysis (TGA) curve of the crystal form I of the p-toluenesulfonate of the compound represented by formula (I) is substantially as shown in FIG. 3.

The present disclosure provides a hydrochloride of a compound represented by formula (I), 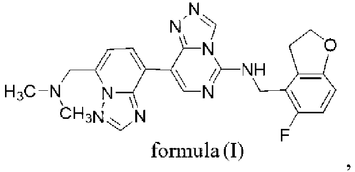 wherein the molar ratio of the compound represented by formula (I) to hydrochloric acid is 1:1.

The present disclosure provides a crystal form I of a hydrochloride of a compound represented by formula (I), wherein an X-ray powder diffraction (XRPD) pattern of the crystal form, measured using Cu-Kα radiation and expressed in terms of 2θ angles, has diffraction peaks at 7.89±0.20°, 11.61±0.20°, 15.89±0.20°, 25.58±0.20°, and 26.45±0.20°, 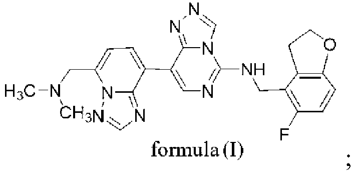 the molar ratio of the compound represented by formula (I) to hydrochloric acid is 1:1.

In a certain embodiment, the X-ray powder diffraction (XRPD) pattern of the crystal form I of the hydrochloride of the compound represented by formula (I), measured using Cu-Kα radiation and expressed in terms of 2θ angles, further has diffraction peaks at one or more of 6.87±0.20°, 16.94±0.20°, 18.72±0.20°, 23.34±0.20°, and 23.96±0.20°.

In a certain embodiment, the X-ray powder diffraction (XRPD) pattern of the crystal form I of the hydrochloride of the compound represented by formula (I), measured using Cu-Kα radiation and expressed in terms of 2θ angles, further has diffraction peaks at one or more of 15.57±0.20°, 28.97±0.20°, 31.46±0.20°, and 39.23±0.20°.

In a certain embodiment, the X-ray powder diffraction (XRPD) pattern of the crystal form I of the hydrochloride of the compound represented by formula (I), measured using Cu-Kα radiation and expressed in terms of 2θ angles, has diffraction peaks at 6.87±0.20°, 7.89±0.20°, 9.25±0.20°, 11.61±0.20°, 12.52±0.20°, 12.98±0.20°, 14.34±0.20°, 15.57±0.20°, 15.89±0.20°, 16.94±0.20°, 17.44±0.20°, 18.72±0.20°, 19.23±0.20°, 20.55±0.20°, 22.22±0.20°, 23.34±0.20°, 23.96±0.20°, 24.46±0.20°, 24.81±0.20°, 25.27±0.20°, 25.58±0.20°, 26.45±0.20°, 27.48±0.20°, 27.92±0.20°, 28.97±0.20°, and 31.46±0.20°.

In a certain embodiment, the X-ray powder diffraction (XRPD) pattern of the crystal form I of the hydrochloride of the compound represented by formula (I), measured using Cu-Kα radiation and expressed in terms of 2θ angles, exhibits diffraction peak positions as shown in the following table:

| No. | 2θ (°) | No. | 2θ (°) |
|---|---|---|---|
| 1 | 6.87 | 24 | 25.58 |
| 2 | 7.89 | 25 | 26.45 |
| 3 | 9.25 | 26 | 27.48 |
| 4 | 11.61 | 27 | 27.92 |
| 5 | 12.52 | 28 | 28.11 |
| 6 | 12.98 | 29 | 28.97 |
| 7 | 13.27 | 30 | 29.34 |
| 8 | 14.34 | 31 | 30.34 |
| 9 | 15.57 | 32 | 30.84 |
| 10 | 15.89 | 33 | 31.46 |
| 11 | 16.94 | 34 | 32.16 |
| 12 | 17.44 | 35 | 32.61 |
| 13 | 17.74 | 36 | 33.10 |
| 14 | 18.72 | 37 | 33.75 |
| 15 | 19.23 | 38 | 34.33 |
| 16 | 20.27 | 39 | 34.63 |
| 17 | 20.55 | 40 | 35.46 |
| 18 | 22.22 | 41 | 35.88 |
| 19 | 23.34 | 42 | 36.76 |
| 20 | 23.96 | 43 | 37.57 |
| 21 | 24.46 | 44 | 38.03 |
| 22 | 24.81 | 45 | 39.23 |
| 23 | 25.27 | | |

In a certain embodiment, the X-ray powder diffraction (XRPD) pattern of the crystal form I of the hydrochloride of the compound represented by formula (I), measured using Cu-Kα radiation and expressed in terms of 2θ angles, exhibits diffraction peak positions and relative intensities as shown in the following table:

| No. | 2θ (°) | Relative intensity (%) | No. | 2θ (°) | Relative intensity (%) |
|---|---|---|---|---|---|
| 1 | 6.87 | 3.8 | 24 | 25.58 | 8.4 |
| 2 | 7.89 | 100.0 | 25 | 26.45 | 30.4 |
| 3 | 9.25 | 0.5 | 26 | 27.48 | 0.3 |
| 4 | 11.61 | 12.7 | 27 | 27.92 | 0.8 |
| 5 | 12.52 | 1.7 | 28 | 28.11 | 0.9 |
| 6 | 12.98 | 0.4 | 29 | 28.97 | 2.2 |
| 7 | 13.27 | 0.1 | 30 | 29.34 | 1.0 |
| 8 | 14.34 | 0.9 | 31 | 30.34 | 0.6 |
| 9 | 15.57 | 2.3 | 32 | 30.84 | 0.4 |
| 10 | 15.89 | 51.2 | 33 | 31.46 | 2.1 |
| 11 | 16.94 | 2.6 | 34 | 32.16 | 0.4 |
| 12 | 17.44 | 1.2 | 35 | 32.61 | 0.2 |
| 13 | 17.74 | 0.2 | 36 | 33.10 | 0.3 |
| 14 | 18.72 | 4.2 | 37 | 33.75 | 0.3 |
| 15 | 19.23 | 0.5 | 38 | 34.33 | 0.2 |
| 16 | 20.27 | 0.3 | 39 | 34.63 | 0.2 |
| 17 | 20.55 | 1.7 | 40 | 35.46 | 0.4 |
| 18 | 22.22 | 1.7 | 41 | 35.88 | 0.1 |
| 19 | 23.34 | 6.2 | 42 | 36.76 | 0.3 |
| 20 | 23.96 | 4.3 | 43 | 37.57 | 0.8 |
| 21 | 24.46 | 1.1 | 44 | 38.03 | 1.7 |
| 22 | 24.81 | 1.5 | 45 | 39.23 | 2.0 |
| 23 | 25.27 | 0.6 | | | |

In a certain embodiment, the X-ray powder diffraction (XRPD) pattern of the crystal form I of the hydrochloride of the compound represented by formula (I), measured using Cu-Kα radiation and expressed in terms of 2θ angles, is substantially as shown in FIG. 4.

In a certain embodiment, a differential scanning calorimetry (DSC) curve of the crystal form I of the hydrochloride of the compound represented by formula (I) shows no endothermic peak below 250±3 °C (e.g., in a range of 40±3 °C to 250±3 °C, such as in a range of 80±3 °C to 250±3 °C).

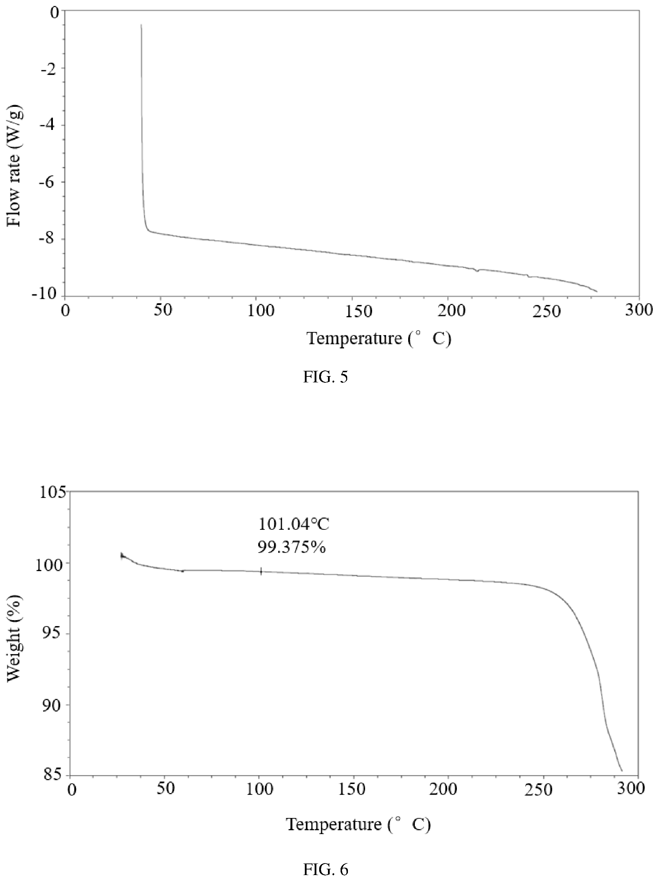

In a certain embodiment, the differential scanning calorimetry (DSC) curve of the crystal form I of the hydrochloride of the compound represented by formula (I) is substantially as shown in FIG. 5.

In a certain embodiment, a thermogravimetric analysis (TGA) curve of the crystal form I of the hydrochloride of the compound represented by formula (I) shows a weight loss of 0.625% at 101.04±3 °C (e.g., in a range of 25±3 °C to 101.04±3 °C).

In a certain embodiment, the thermogravimetric analysis (TGA) curve of the crystal form I of the hydrochloride of the compound represented by formula (I) is substantially as shown in FIG. 6.

The present disclosure provides a crystal form B of a compound represented by formula (I), wherein an X-ray powder diffraction (XRPD) pattern of the crystal form, measured using Cu-Kα radiation and expressed in terms of 2θ angles, has diffraction peaks at 8.39±0.20°, 9.56±0.20°, 16.78±0.20°, 17.19±0.20°, 22.22±0.20°, and 25.15±0.20°,

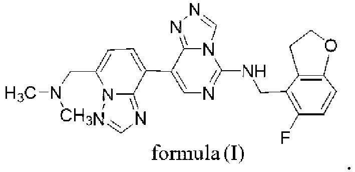

In a certain embodiment, the X-ray powder diffraction (XRPD) pattern of the crystal form B of the compound represented by formula (I), measured using Cu-Kα radiation and expressed in terms of 2θ angles, further has diffraction peaks at one or more of 11.78±0.20°, 13.35±0.20°, 19.21±0.20°, 20.14±0.20°, 26.13±0.20°, and 31.12±0.20°.

In a certain embodiment, the X-ray powder diffraction (XRPD) pattern of the crystal form B of the compound represented by formula (I), measured using Cu-Kα radiation and expressed in terms of 2θ angles, further has diffraction peaks at one or more of 6.65±0.20°, 12.91±0.20°, 14.00±0.20°, 16.17±0.20°, 27.77±0.20°, and 27.98±0.20°.

In a certain embodiment, the X-ray powder diffraction (XRPD) pattern of the crystal form B of the compound represented by formula (I), measured using Cu-Kα radiation and expressed in terms of 2θ angles, has diffraction peaks at 6.65±0.20°, 8.39±0.20°, 9.56±0.20°, 11.78±0.20°, 12.91±0.20°, 13.35±0.20°, 14.00±0.20°, 14.63±0.20°, 16.17±0.20°, 16.78±0.20°, 17.19±0.20°, 19.21±0.20°, 20.14±0.20°, 22.22±0.20°, 23.39±0.20°, 23.85±0.20°, 24.14±0.20°, 25.15±0.20°, 26.13±0.20°, 26.82±0.20°, 27.19±0.20°, 27.77±0.20°, 27.98±0.20°, 28.34±0.20°, 30.58±0.20°, 31.12±0.20°, 33.08±0.20°, and 34.26±0.20°.

In a certain embodiment, the X-ray powder diffraction (XRPD) pattern of the crystal form B of the compound represented by formula (I), measured using Cu-Kα radiation and expressed in terms of 2θ angles, exhibits diffraction peak positions as shown in the following table:

| No. | 2θ (°) | No. | 2θ (°) |
|---|---|---|---|
| 1 | 6.65 | 29 | 25.15 |
| 2 | 7.95 | 30 | 25.65 |
| 3 | 8.39 | 31 | 26.13 |
| 4 | 9.56 | 32 | 26.82 |
| 5 | 11.78 | 33 | 27.19 |
| 6 | 12.55 | 34 | 27.77 |
| 7 | 12.91 | 35 | 27.98 |
| 8 | 13.35 | 36 | 28.34 |
| 9 | 14.00 | 37 | 28.99 |
| 10 | 14.63 | 38 | 29.57 |
| 11 | 15.13 | 39 | 30.25 |
| 12 | 15.54 | 40 | 30.58 |
| 13 | 16.17 | 41 | 31.12 |
| 14 | 16.78 | 42 | 31.74 |
| 15 | 17.19 | 43 | 32.51 |
| 16 | 18.03 | 44 | 33.08 |
| 17 | 18.54 | 45 | 33.87 |
| 18 | 19.21 | 46 | 34.26 |
| 19 | 20.14 | 47 | 34.95 |
| 20 | 20.54 | 48 | 35.40 |
| 21 | 21.59 | 49 | 35.84 |
| 22 | 22.22 | 50 | 36.80 |
| 23 | 22.48 | 51 | 37.17 |
| 24 | 22.99 | 52 | 37.61 |
| 25 | 23.39 | 53 | 37.69 |
| 26 | 23.85 | 54 | 39.00 |
| 27 | 24.14 | 55 | 39.25 |
| 28 | 24.87 | 56 | 39.70 |

In a certain embodiment, the X-ray powder diffraction (XRPD) pattern of the crystal form B of the compound represented by formula (I), measured using Cu-Kα radiation and expressed in terms of 2θ angles, exhibits diffraction peak positions and relative intensities as shown in the following table:

| No. | 2θ (°) | Relative intensity | No. | 2θ (°) | Relative intensity |
|---|---|---|---|---|---|
| 1 | 6.65 | 18.0 % | 29 | 25.15 | 66.1 % |
| 2 | 7.95 | 1.4 % | 30 | 25.65 | 6.5 % |
| 3 | 8.39 | 95.1 % | 31 | 26.13 | 35.5 % |
| 4 | 9.56 | 60.6 % | 32 | 26.82 | 7.6 % |
| 5 | 11.78 | 33.8 % | 33 | 27.19 | 7.4 % |
| 6 | 12.55 | 1.0 % | 34 | 27.77 | 11.8 % |
| 7 | 12.91 | 15.7 % | 35 | 27.98 | 10.3 % |
| 8 | 13.35 | 34.6 % | 36 | 28.34 | 9.7 % |
| 9 | 14.00 | 16.1 % | 37 | 28.99 | 0.5 % |
| 10 | 14.63 | 5.9 % | 38 | 29.57 | 2.5 % |
| 11 | 15.13 | 0.3 % | 39 | 30.25 | 1.7 % |
| 12 | 15.54 | 0.4 % | 40 | 30.58 | 7.1 % |
| 13 | 16.17 | 11.1 % | 41 | 31.12 | 20.7 % |
| 14 | 16.78 | 100.0 % | 42 | 31.74 | 0.3 % |
| 15 | 17.19 | 57.9 % | 43 | 32.51 | 3.0 % |
| 16 | 18.03 | 2.9 % | 44 | 33.08 | 8.9 % |
| 17 | 18.54 | 0.9 % | 45 | 33.87 | 0.9 % |
| 18 | 19.21 | 33.7 % | 46 | 34.26 | 8.4 % |
| 19 | 20.14 | 22.5 % | 47 | 34.95 | 1.4 % |
| 20 | 20.54 | 0.8 % | 48 | 35.40 | 0.6 % |
| 21 | 21.59 | 0.2 % | 49 | 35.84 | 0.6 % |
| 22 | 22.22 | 77.8 % | 50 | 36.80 | 0.3 % |
| 23 | 22.48 | 5.6 % | 51 | 37.17 | 0.5 % |
| 24 | 22.99 | 0.6 % | 52 | 37.61 | 3.9 % |
| 25 | 23.39 | 7.2 % | 53 | 37.69 | 2.9 % |
| 26 | 23.85 | 8.1 % | 54 | 39.00 | 0.5 % |
| 27 | 24.14 | 6.0 % | 55 | 39.25 | 1.0 % |
| 28 | 24.87 | 4.7 % | 56 | 39.70 | 1.9 % |

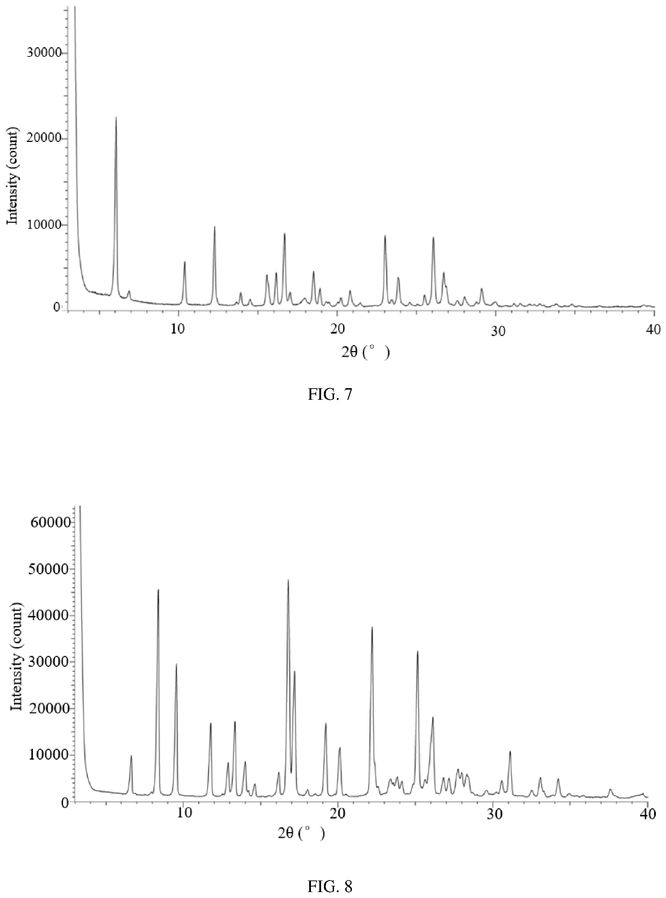

In a certain embodiment, the X-ray powder diffraction (XRPD) pattern of the crystal form B of the compound represented by formula (I), measured using Cu-Kα radiation and expressed in terms of 2θ angles, is substantially as shown in FIG. 8.

In a certain embodiment, a differential scanning calorimetry (DSC) curve of the crystal form B of the compound represented by formula (I) has an endothermic peak onset at 213.77±3 °C, and/or the differential scanning calorimetry (DSC) curve of the crystal form B of the compound represented by formula (I) has a peak temperature of 214.71±3 °C.

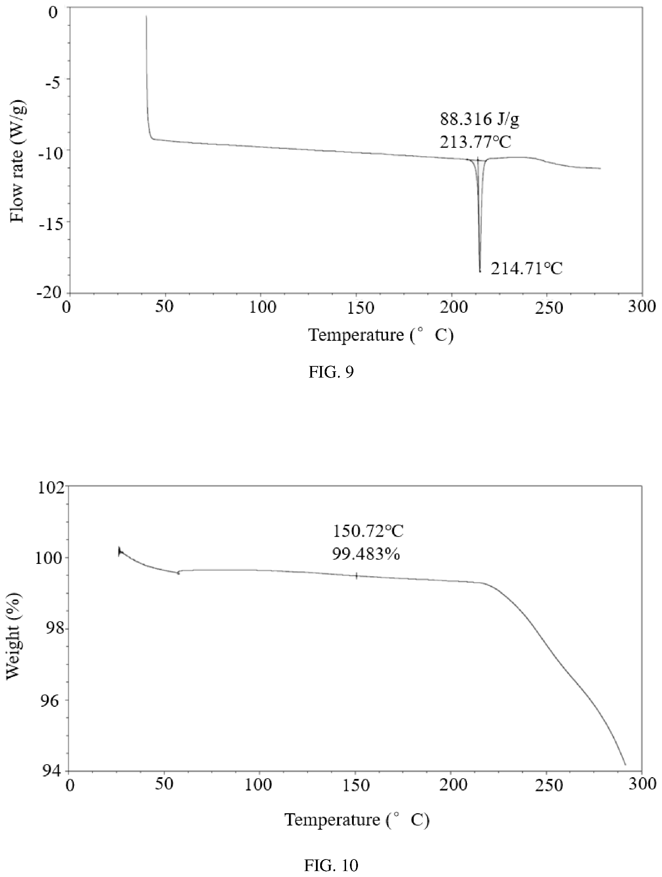

In a certain embodiment, the differential scanning calorimetry (DSC) curve of the crystal form B of the compound represented by formula (I) is substantially as shown in FIG. 9.

In a certain embodiment, a thermogravimetric analysis (TGA) curve of the crystal form B of the compound represented by formula (I) shows a weight loss of 0.517% at 150.72±3 °C (e.g., in a range of 25±3 °C to 150.72±3 °C).

In a certain embodiment, the thermogravimetric analysis (TGA) curve of the crystal form B of the compound represented by formula (I) shows that the crystal form undergoes decomposition above 210±3 °C.

In a certain embodiment, the thermogravimetric analysis (TGA) curve of the crystal form B of the compound represented by formula (I) is substantially as shown in FIG. 10.

The present disclosure further provides a preparation method for the crystal form I of the p-toluenesulfonate of the compound represented by formula (I), which comprises the following step: crystallizing a mixture of p-toluenesulfonic acid, the compound represented by formula (I), and a solvent to obtain the crystal form I of the p-toluenesulfonate of the compound represented by formula (I), wherein the solvent is an alcohol solvent or a mixed solvent of an alcohol solvent and water.

In a certain embodiment, in the preparation method for the crystal form I of the p-toluenesulfonate of the compound represented by formula (I), the alcohol solvent is a C₁-C₃ alcohol solvent, for example, methanol, ethanol, n-propanol, or isopropanol, such as ethanol.

In a certain embodiment, the mixed solvent of the alcohol solvent and water is a mixed solvent of ethanol and water in a volume ratio of 3:1.

In a certain embodiment, in the preparation method for the crystal form I of the p-toluenesulfonate of the compound represented by formula (I), the p-toluenesulfonic acid is conventional in the art, for example, p-toluenesulfonic acid monohydrate.

In a certain embodiment, in the preparation method for the crystal form I of the p-toluenesulfonate of the compound represented by formula (I), the crystallization is a conventional crystallization operation in the art, for example, cooling crystallization.

In a certain embodiment, the preparation method for the crystal form I of the p-toluenesulfonate of the compound represented by formula (I) further comprises filtering and drying to obtain the crystal form I of the p-toluenesulfonate of the compound represented by formula (I).

Preferably, in the preparation method for the crystal form I of the p-toluenesulfonate of the compound represented by formula (I), the drying is drying *in vacuo* under reduced pressure at 40-60 °C.

The present disclosure further provides a preparation method for the crystal form I of the hydrochloride of the compound represented by formula (I), which comprises the following step: crystallizing a mixture of hydrochloric acid, the compound represented by formula (I), and a solvent to obtain the crystal form I of the hydrochloride of the compound represented by formula (I), wherein the solvent is an alcohol solvent or a mixed solvent of an alcohol solvent and water.

In a certain embodiment, in the preparation method for the crystal form I of the hydrochloride of the compound represented by formula (I), the alcohol solvent is a C₁-C₃ alcohol solvent, for example, methanol, ethanol, n-propanol, or isopropanol, such as ethanol.

In a certain embodiment, in the preparation method for the crystal form I of the hydrochloride of the compound represented by formula (I), the mixed solvent of the alcohol solvent and water is a mixed solvent of ethanol and water in a volume ratio of 3:1.

In a certain embodiment, the preparation method for the crystal form I of the hydrochloride of the compound represented by formula (I) comprises the following steps:

mixing the compound represented by formula (I), hydrochloric acid, and a solvent, crystallizing, filtering, and drying to obtain the crystal form I of the hydrochloride of the compound represented by formula (I).

Preferably, the crystallization, the filtration, and the drying are performed as described in any one of the embodiments of the present disclosure.

The present disclosure further provides a preparation method for the crystal form B of the compound represented by formula (I), which comprises the following step: crystallizing a mixture of the compound represented by formula (I) and a solvent to obtain the crystal form B of the compound represented by formula (I), wherein the solvent is an ester solvent.

In a certain embodiment, in the preparation method for the crystal form B of the compound represented by formula (I), the ester solvent is ethyl acetate.

In a certain embodiment, in the preparation method for the crystal form B of the compound represented by formula (I), the crystallization is slurrying.

In a certain embodiment, in the preparation method for the crystal form B of the compound represented by formula (I), the compound represented by formula (I) is a crystal form A of the compound represented by formula (I).

The present disclosure further provides a pharmaceutical composition, which comprises a substance Z and one or more pharmaceutically acceptable auxiliary materials, wherein the substance Z is the p-toluenesulfonate of the compound represented by formula (I) described above, the hydrochloride of the compound represented by formula (I) described above, the crystal form I of the p-toluenesulfonate of the compound represented by formula (I) described above, the crystal form I of the hydrochloride of the compound represented by formula (I) described above, or the crystal form B of the compound represented by formula (I) described above. The pharmaceutical composition comprises, as an active ingredient, the p-toluenesulfonate of the compound represented by formula (I) described above, the hydrochloride of the compound represented by formula (I) described above, the crystal form I of the p-toluenesulfonate of the compound represented by formula (I) described above, the crystal form I of the hydrochloride of the compound represented by formula (I) described above, or the crystal form B of the compound represented by formula (I) described above.

The present disclosure further provides use of a substance Z or the pharmaceutical composition described above in the manufacture of a medicament for preventing and/or treating a disease associated with the mechanism of action of an EED protein and/or a PRC2 protein complex, wherein the substance Z is the p-toluenesulfonate of the compound represented by formula (I) described above, the hydrochloride of the compound represented by formula (I) described above, the crystal form I of the p-toluenesulfonate of the compound represented by formula (I) described above, the crystal form I of the hydrochloride of the compound represented by formula (I) described above, or the crystal form B of the compound represented by formula (I) described above.

In a certain embodiment, the disease is a tumor (e.g., a malignant tumor), for example, lymphoma, leukemia, multiple myeloma, mesothelioma, gastric cancer, malignant rhabdoid tumor, liver cancer, prostate cancer, breast cancer, brain tumor including neuroblastoma, glioma, glioblastoma and astrocytoma, cervical cancer, colon cancer, melanoma, endometrial cancer, esophageal cancer, head and neck cancer, lung cancer, nasopharyngeal cancer, ovarian cancer, pancreatic cancer, renal cancer, rectal cancer, thyroid cancer, parathyroid tumor, uterine tumor, and soft tissue tumor, wherein the lymphoma is preferably diffuse large B-cell lymphoma, follicular lymphoma, or non-Hodgkin's lymphoma.

### Terms and Definitions:

It should be noted that in the X-ray powder diffraction pattern, the positions and relative intensities of diffraction peaks may vary due to factors such as the measuring instrument and the measurement method/conditions. For any specific crystal form, the position of a peak may have an error, and the measurement error of the 2θ value is, for example, ±0.2°. Therefore, this error should be taken into account when identifying each crystal form, and crystal forms within this margin of error also fall within the scope of the present application.

It should be noted that, for the same crystal form, the positions of thermal events (e.g., endothermic peaks and exothermic peaks) in the DSC curve may vary due to factors such as the measuring instrument and the measurement method/conditions. For any specific crystal form, the position of an endothermic peak may have an error of ±5 °C, ±3 °C, or ±2 °C. Therefore, this error should be taken into account when identifying each crystal form, and crystal forms within this margin of error also fall within the scope of the present application.

It should be noted that, for the same crystal form, the temperature at which weight loss occurs in the TGA curve may vary due to factors such as the measuring instrument and the measurement method/conditions. For any specific crystal form, the temperature at which weight loss occurs may have an error of ±5 °C, ±3 °C, or ±2 °C. Therefore, this error should be taken into account when identifying each crystal form, and crystal forms within this margin of error also fall within the scope of the present application.

In the present application, "pharmaceutical composition" refers to a formulation of the compound of the present disclosure with a medium generally acceptable in the art for delivering a biologically active compound to a mammal (e.g., a human). The medium includes a pharmaceutically acceptable carrier. The purpose of the pharmaceutical composition is to facilitate administration to an organism, promote absorption of the active ingredient, and thereby exert biological activity. The term "pharmaceutically acceptable" as used herein refers to a substance (e.g., carrier or diluent) that does not affect the biological activity or properties of the compound of the present disclosure and is relatively non-toxic, i.e., the substance can be administered to an individual without causing an adverse biological response or interacting in an undesirable manner with any of the components contained in the composition. In the present application, "pharmaceutically acceptable auxiliary material" includes, but is not limited to, any adjuvant, carrier, excipient, glidant, sweetener, diluent, preservative, dye/colorant, flavoring agent, surfactant, wetting agent, dispersant, suspending agent, stabilizer, isotonic agent, solvent, or emulsifier that is approved by the relevant governmental regulatory agency for human or livestock use.

The terms "prevent", "preventing", and "prevention" as used herein include reducing the possibility of the occurrence or exacerbation of a disease or disorder in a patient.

The term "treatment" and other similar synonyms used herein include the following meanings: (i) preventing the occurrence of a disease or disorder in a mammal, particularly when the mammal is predisposed to the disease or disorder but has not yet been diagnosed as having the disease or disorder; (ii) inhibiting the disease or disorder, i.e., arresting its development; (iii) alleviating the disease or disorder, i.e., causing regression of the state of the disease or disorder; or (iv) relieving symptoms caused by the disease or disorder.

As used herein and unless otherwise specified, the term "auxiliary material" refers to those auxiliary materials widely used in the field of pharmaceutical production. The auxiliary materials are primarily used to provide a safe, stable and functional pharmaceutical composition, and may also provide methods for dissolving the active ingredient at a desired rate or for promoting effective absorption of the active ingredient after administration of the composition to a subject. The auxiliary materials may be inert fillers, or may provide certain functions such as stabilizing the overall pH of the composition or preventing degradation of the active ingredient of the composition.

As used herein, the term "room temperature" generally refers to 10 °C to 35 °C, for example, 25 °C, unless otherwise specified.

As used herein, the term "stirring" refers to a stirring operation performed using a conventional method in the art, such as magnetic stirring or mechanical stirring, at a stirring speed of 50-1800 rpm, wherein the magnetic stirring is performed at 200-1500 rpm, preferably 300-1000 rpm, and the mechanical stirring is preferably performed at 100-300 rpm.

Unless otherwise stated, the term "more" (e.g., more items/positions/types) as used herein refers to, for example, 2, 3, 4, 5, or 6 items/positions/types.

Without departing from the general knowledge in the art, the above various preferred conditions may be combined arbitrarily to obtain various preferred examples of the present disclosure.

The reagents and starting materials used in the present disclosure are all commercially available.

The positive and progressive effects of the present disclosure are as follows: the crystal forms of the salts of the compound represented by formula (I) provided by the present disclosure have relatively good solubility and physicochemical stability, and exhibit favorable stability under high temperature and high humidity conditions; moreover, the crystal forms maintain good stability under grinding, compression, and heating conditions; compared with the free base, the salts of the compound represented by formula (I) exhibit higher AUC and Cₘₐₓ, shorter time to peak *in vivo*, and greater exposure, thus possessing excellent pharmacokinetic properties.

### BRIEF DESCRIPTION OF THE DRAWINGS

FIG. 1 shows the XRPD pattern of the crystal form I of the p-toluenesulfonate of the compound represented by formula (I).
FIG. 2 shows the DSC profile of the crystal form I of the p-toluenesulfonate of the compound represented by formula (I).
FIG. 3 shows the TGA profile of the crystal form I of the p-toluenesulfonate of the compound represented by formula (I).
FIG. 4 shows the XRPD pattern of the crystal form I of the hydrochloride of the compound represented by formula (I).
FIG. 5 shows the DSC profile of the crystal form I of the hydrochloride of the compound represented by formula (I).
FIG. 6 shows the TGA profile of the crystal form I of the hydrochloride of the compound represented by formula (I).
FIG. 7 shows the XRPD pattern of the crystal form A of the compound represented by formula (I).
FIG. 8 shows the XRPD pattern of the crystal form B of the compound represented by formula (I).
FIG. 9 shows the DSC profile of the crystal form B of the compound represented by formula (I).
FIG. 10 shows the TGA profile of the crystal form B of the compound represented by formula (I).

### DETAILED DESCRIPTION

The present disclosure is further illustrated by the following examples; however, these examples should not be construed as limiting the present disclosure. Experimental procedures without specified conditions in the following examples were performed according to conventional methods and conditions, or as directed in product instructions.

Testing instruments used in experiments

### 1. X-ray diffraction pattern

Instrument model: Bruker D8 ADVANCE X-ray powder diffractometer
Radiation: monochromatic Cu-Kα radiation (wavelength = 1.5418)
Scan mode: θ/2θ, scan range: 3-60°
Voltage: 40 kV, current: 40 mA

### 2. DSC profile

Instrument model: TA Discovery DSC 250
Purge gas: nitrogen
Ramp rate: 10 °C/min
Temperature range: 40 °C to 285 °C

### 3. TGA profile

Instrument model: TA Discovery TGA 550
Purge gas: nitrogen
Ramp rate: 10 °C/min
Temperature range: room temperature to 300 °C

### Example 1: Preparation of Crystal Form I of p-Toluenesulfonate of Compound Represented by Formula (I)

300 mg (0.65 mmol) of the compound represented by formula (I) (the compound was prepared according to the method disclosed in Example 115 of WO2021032004) was taken and added into a 25 mL single-neck flask, and 12 mL of 75% ethanol (v/v) and 147 mg (0.77 mmol) of p-toluenesulfonic acid monohydrate were added. The mixture was heated for dissolution and then cooled to 20 °C for crystallization for 2 h. The precipitate was collected by filtration, rinsed with 75% ethanol, and dried *in vacuo* at 50 °C to give a solid (375 mg, yield: 90.92%). The resulting solid was identified by XRPD, DSC, and TGA as a crystal form I of a p-toluenesulfonate. The p-toluenesulfonic acid content was determined by HPLC (with reference to the method described in General Chapter 0512, Volume IV of the Chinese Pharmacopoeia (2020 Edition)), and the results indicated that the molar ratio of the compound represented by formula (I) to p-toluenesulfonic acid was 1:1.

**Table 1. XRPD peak list of crystal form I of p-toluenesulfonate**

| No. | 2θ (°) | d value (Å) | Relative intensity (%) | No. | 2θ (°) | d value (Å) | Relative intensity (%) |
|---|---|---|---|---|---|---|---|
| 1 | 5.98 | 14.77075 | 0.3 | 45 | 34.56 | 2.59325 | 0.5 |
| 2 | 6.63 | 13.32415 | 93.0 | 46 | 34.91 | 2.56815 | 0.3 |
| 3 | 8.82 | 10.01717 | 0.2 | 47 | 35.11 | 2.55395 | 0.2 |
| 4 | 10.87 | 8.13092 | 3.7 | 48 | 35.68 | 2.51433 | 0.5 |
| 5 | 11.91 | 7.42788 | 26.2 | 49 | 36.18 | 2.48111 | 0.6 |
| 6 | 13.24 | 6.68054 | 100.0 | 50 | 36.75 | 2.44375 | 0.3 |
| 7 | 14.03 | 6.30619 | 11.5 | 51 | 36.99 | 2.42853 | 0.3 |
| 8 | 14.29 | 6.19505 | 3.6 | 52 | 37.49 | 2.39728 | 0.4 |
| 9 | 15.71 | 5.63817 | 12.9 | 53 | 37.95 | 2.36906 | 0.1 |
| 10 | 16.37 | 5.40949 | 0.2 | 54 | 38.28 | 2.3491 | 0.1 |
| 11 | 17.24 | 5.14088 | 6.7 | 55 | 38.67 | 2.32631 | 0.5 |
| 12 | 17.70 | 5.00562 | 2.5 | 56 | 39.19 | 2.29665 | 0.1 |
| 13 | 18.14 | 4.88546 | 12.8 | 57 | 40.40 | 2.23065 | 2.1 |
| 14 | 18.99 | 4.66927 | 2.1 | 58 | 40.91 | 2.20431 | 0.5 |
| 15 | 19.76 | 4.48991 | 0.1 | 59 | 41.57 | 2.17075 | 0.3 |
| 16 | 20.14 | 4.40625 | 0.2 | 60 | 41.99 | 2.15011 | 1.5 |
| 17 | 20.44 | 4.34084 | 0.2 | 61 | 42.79 | 2.11169 | 0.1 |
| 18 | 20.86 | 4.25598 | 1.0 | 62 | 43.29 | 2.08829 | 0.3 |
| 19 | 21.24 | 4.18017 | 1.9 | 63 | 43.98 | 2.05722 | 0.5 |
| 20 | 21.63 | 4.10507 | 3.2 | 64 | 44.82 | 2.02068 | 0.6 |
| 21 | 21.92 | 4.05116 | 5.8 | 65 | 45.82 | 1.97892 | 0.1 |
| 22 | 22.54 | 3.9422 | 5.4 | 66 | 46.66 | 1.94497 | 0.0 |
| 23 | 23.26 | 3.82127 | 0.3 | 67 | 47.19 | 1.92439 | 1.6 |
| 24 | 23.55 | 3.7754 | 0.7 | 68 | 47.45 | 1.9147 | 0.3 |
| 25 | 24.06 | 3.69664 | 1.4 | 69 | 48.19 | 1.88693 | 0.4 |
| 26 | 24.79 | 3.58867 | 5.3 | 70 | 49.04 | 1.85598 | 0.6 |
| 27 | 25.06 | 3.5503 | 6.3 | 71 | 49.64 | 1.83507 | 0.2 |
| 28 | 26.03 | 3.42056 | 1.8 | 72 | 50.02 | 1.82203 | 0.3 |
| 29 | 26.63 | 3.34473 | 8.8 | 73 | 50.56 | 1.80383 | 0.2 |
| 30 | 27.27 | 3.26774 | 0.5 | 74 | 50.71 | 1.79899 | 0.2 |
| 31 | 27.49 | 3.24214 | 0.9 | 75 | 51.59 | 1.77013 | 0.2 |
| 32 | 28.02 | 3.18166 | 1.1 | 76 | 52.00 | 1.7573 | 0.1 |
| 33 | 28.32 | 3.14842 | 0.9 | 77 | 52.39 | 1.74514 | 0.1 |
| 34 | 28.68 | 3.1104 | 0.3 | 78 | 53.51 | 1.71126 | 0.1 |
| 35 | 29.23 | 3.05315 | 0.1 | 79 | 54.70 | 1.67679 | 0.2 |
| 36 | 29.84 | 2.99169 | 0.3 | 80 | 55.04 | 1.66699 | 0.4 |
| 37 | 30.20 | 2.95739 | 0.4 | 81 | 55.81 | 1.64585 | 0.1 |
| 38 | 30.71 | 2.90939 | 1.1 | 82 | 56.39 | 1.63036 | 0.1 |
| 39 | 31.13 | 2.87071 | 0.3 | 83 | 56.96 | 1.61536 | 0.1 |
| 40 | 31.59 | 2.83039 | 1.0 | 84 | 57.76 | 1.59482 | 0.2 |
| 41 | 32.30 | 2.76927 | 1.1 | 85 | 58.41 | 1.57859 | 0.1 |
| 42 | 32.79 | 2.72931 | 0.4 | 86 | 58.91 | 1.56651 | 0.0 |
| 43 | 33.21 | 2.6953 | 3.2 | 87 | 59.45 | 1.55344 | 0.1 |
| 44 | 33.90 | 2.64233 | 0.8 | | | | |

The X-ray diffraction pattern of the crystal form I of the p-toluenesulfonate of the compound represented by formula (I) is shown in FIG. 1. It had characteristic absorption peaks at approximately 6.63°, 10.87°, 11.91°, 13.24°, 14.03°, 14.29°, 15.71°, 17.24°, 18.14°, 22.54°, 24.79°, 25.06°, and 26.63°. It had diffraction peaks as shown in Table 1. The DSC profile is shown in FIG. 2, showing a sharp endothermic peak at 269.39 °C, with an endothermic peak onset at 266.75 °C and an enthalpy value of 77.660 J/g. The TGA profile is shown in FIG. 3, showing substantially no weight loss below 100 °C, substantially no weight loss below 150.48 °C, and gradual melting and decomposition above 250 °C.

### Example 2: Preparation of Crystal Form I of Hydrochloride of Compound Represented by Formula (I)

100 mg (0.22 mmol) of the compound represented by formula (I) was taken and added into a 25 mL single-neck flask, and 4 mL of a 75% aqueous ethanol solution (v/v) and 10 mg (0.27 mmol) of hydrochloric acid were added. The mixture was heated and stirred for dissolution and then cooled to room temperature for crystallization for 24 h. The precipitate was collected by filtration, rinsed with an aqueous ethanol solution, and dried *in vacuo* at 50 °C to give a solid (102 mg, yield: 94.50%). The resulting solid was identified by XRPD, DSC, and TGA as a crystal form I of a hydrochloride of the compound represented by formula (I). The hydrochloric acid content was determined by titration, and the results indicated that the molar ratio of the compound represented by formula (I) to hydrochloric acid was 1:1.

**Table 2. XRPD peak list of crystal form I of hydrochloride**

| No. | 2θ (°) | d value (Å) | Relative intensity (%) | No. | 2θ (°) | d value (Å) | Relative intensity (%) |
|---|---|---|---|---|---|---|---|
| 1 | 6.87 | 12.86339 | 3.8 | 24 | 25.58 | 3.47927 | 8.4 |
| 2 | 7.89 | 11.19074 | 100.0 | 25 | 26.45 | 3.3666 | 30.4 |
| 3 | 9.25 | 9.55248 | 0.5 | 26 | 27.48 | 3.24294 | 0.3 |
| 4 | 11.61 | 7.61568 | 12.7 | 27 | 27.92 | 3.19275 | 0.8 |
| 5 | 12.52 | 7.06675 | 1.7 | 28 | 28.11 | 3.17152 | 0.9 |
| 6 | 12.98 | 6.81322 | 0.4 | 29 | 28.97 | 3.07974 | 2.2 |
| 7 | 13.27 | 6.66727 | 0.1 | 30 | 29.34 | 3.04186 | 1.0 |
| 8 | 14.34 | 6.17113 | 0.9 | 31 | 30.34 | 2.94411 | 0.6 |
| 9 | 15.57 | 5.68662 | 2.3 | 32 | 30.84 | 2.8969 | 0.4 |
| 10 | 15.89 | 5.57417 | 51.2 | 33 | 31.46 | 2.84129 | 2.1 |
| 11 | 16.94 | 5.2312 | 2.6 | 34 | 32.16 | 2.78127 | 0.4 |
| 12 | 17.44 | 5.08215 | 1.2 | 35 | 32.61 | 2.74353 | 0.2 |
| 13 | 17.74 | 4.99645 | 0.2 | 36 | 33.10 | 2.7042 | 0.3 |
| 14 | 18.72 | 4.73553 | 4.2 | 37 | 33.75 | 2.6538 | 0.3 |
| 15 | 19.23 | 4.61086 | 0.5 | 38 | 34.33 | 2.60987 | 0.2 |
| 16 | 20.27 | 4.37776 | 0.3 | 39 | 34.63 | 2.58829 | 0.2 |
| 17 | 20.55 | 4.31914 | 1.7 | 40 | 35.46 | 2.52977 | 0.4 |
| 18 | 22.22 | 3.99763 | 1.7 | 41 | 35.88 | 2.5006 | 0.1 |
| 19 | 23.34 | 3.80847 | 6.2 | 42 | 36.76 | 2.44301 | 0.3 |
| 20 | 23.96 | 3.71116 | 4.3 | 43 | 37.57 | 2.39184 | 0.8 |
| 21 | 24.46 | 3.63578 | 1.1 | 44 | 38.03 | 2.36418 | 1.7 |
| 22 | 24.81 | 3.58537 | 1.5 | 45 | 39.23 | 2.29463 | 2.0 |
| 23 | 25.27 | 3.52103 | 0.6 | | | | |

The X-ray diffraction pattern of the crystal form I of the hydrochloride of the compound represented by formula (I) is shown in FIG. 4. It had characteristic absorption peaks at 2θ angles of 6.87°, 7.89°, 9.25°, 11.61°, 12.52°, 12.98°, 14.34°, 15.57°, 15.89°, 16.94°, 17.44°, 18.72°, 19.23°, 20.55°, 22.22°, 23.34°, 23.96°, 24.46°, 24.81°, 25.27°, 25.58°, 26.45°, 27.48°, 27.92°, 28.97°, and 31.46°, wherein the error range of the 2θ angle for each characteristic peak was ±0.2°. It had diffraction peaks as shown in Table 2. The DSC profile is shown in FIG. 5, showing no melting endothermic peak below 250 °C. The TGA profile is shown in FIG. 6, showing nearly no weight loss below 100 °C, and a weight loss of about 0.625% (remaining weight: 99.375%) at 101.04 °C.

### Example 3: Preparation of Crystal Form A of Compound Represented by Formula (I)

The compound represented by formula (I) was prepared according to the method disclosed in Example 115 of WO2021032004. After preparation, the resulting product was concentrated to give a crystalline compound. The resulting solid was identified by XRPD and TGA as a crystal form A of the compound represented by formula (I).

**Table 3. XRPD peak list of crystal form A of compound represented by formula (I)**

| No. | 2θ (°) | d value (Å) | Relative intensity | No. | 2θ (°) | d value (Å) | Relative intensity |
|---|---|---|---|---|---|---|---|
| 1 | 6.11 | 14.46589 | 100.0 % | 24 | 25.13 | 3.54042 | 0.8 % |
| 2 | 6.94 | 12.73385 | 3.5 % | 25 | 25.57 | 3.48048 | 7.0 % |
| 3 | 10.44 | 8.46571 | 22.9 % | 26 | 26.14 | 3.40685 | 38.1 % |
| 4 | 12.31 | 7.18756 | 54.8 % | 27 | 26.78 | 3.32689 | 17.5 % |
| 5 | 13.73 | 6.4438 | 1.7 % | 28 | 27.64 | 3.22449 | 2.8 % |
| 6 | 13.97 | 6.33343 | 6.4 % | 29 | 28.09 | 3.17409 | 8.0 % |
| 7 | 14.58 | 6.06992 | 3.7 % | 30 | 28.97 | 3.07935 | 3.0 % |
| 8 | 15.68 | 5.64599 | 21.0 % | 31 | 29.19 | 3.05725 | 15.7 % |
| 9 | 16.20 | 5.46825 | 22.6 % | 32 | 30.04 | 2.97259 | 2.9 % |
| 10 | 16.72 | 5.2967 | 42.7 % | 33 | 31.20 | 2.86418 | 2.6 % |
| 11 | 17.09 | 5.18558 | 8.8 % | 34 | 31.60 | 2.82886 | 1.8 % |
| 12 | 17.97 | 4.93364 | 22.2 % | 35 | 32.20 | 2.778 | 1.0 % |
| 13 | 18.55 | 4.7799 | 26.4 % | 36 | 32.43 | 2.75817 | 0.8 % |
| 14 | 18.94 | 4.68156 | 11.3 % | 37 | 32.86 | 2.72379 | 1.7 % |
| 15 | 19.41 | 4.57008 | 2.5 % | 38 | 33.62 | 2.66339 | 1.1 % |
| 16 | 20.30 | 4.37127 | 4.6 % | 39 | 33.83 | 2.64779 | 1.7 % |
| 17 | 20.89 | 4.24861 | 8.1 % | 40 | 34.54 | 2.59442 | 0.4 % |
| 18 | 21.53 | 4.12335 | 3.1 % | 41 | 34.85 | 2.57209 | 2.3 % |
| 19 | 22.49 | 3.94949 | 0.6 % | 42 | 35.33 | 2.53877 | 0.7 % |
| 20 | 23.09 | 3.84959 | 64.3 % | 43 | 36.60 | 2.45321 | 1.0 % |
| 21 | 23.51 | 3.78177 | 4.1 % | 44 | 37.69 | 2.38452 | 0.8 % |
| 22 | 23.92 | 3.71666 | 26.3 % | 45 | 38.56 | 2.33285 | 0.7 % |
| 23 | 24.64 | 3.61054 | 1.6 % | 46 | 39.39 | 2.28564 | 1.2 % |

The XRPD data of the crystal form A of the compound represented by formula (I) are shown in Table 3, and the corresponding XRPD pattern is shown in FIG. 7. It had characteristic absorption peaks at diffraction 2θ angles of 6.11°, 6.94°, 10.44°, 12.31°, 13.97°, 14.58°, 15.68°, 16.20°, 16.72°, 17.09°, 17.97°, 18.55°, 18.94°, 19.41°, 20.30°, 20.89°, 21.53°, 23.09°, 23.92°, 25.57°, 26.14°, 26.78°, 28.09°, and 29.19°, wherein the error range of the 2θ angle for each characteristic peak was ±0.2°. The TGA profile showed a weight loss of about 5.92% below 100 °C.

### Example 4: Crystal Form B of Free Base of Compound Represented by Formula (I)

100 mg of the solid of the crystal form A of the compound represented by formula (I) was taken and added to 5 mL of ethyl acetate for a slurry experiment to give a crystal form B of the compound represented by formula (I).

**Table 4. XRPD peak list of crystal form B**

| No. | 2θ (°) | d value (Å) | Relative intensity | No. | 2θ (°) | d value (Å) | Relative intensity |
|---|---|---|---|---|---|---|---|
| 1 | 6.65 | 13.27407 | 18.0 % | 29 | 25.15 | 3.53786 | 66.1 % |
| 2 | 7.95 | 11.10697 | 1.4 % | 30 | 25.65 | 3.47033 | 6.5 % |
| 3 | 8.39 | 10.52562 | 95.1 % | 31 | 26.13 | 3.40751 | 35.5 % |
| 4 | 9.56 | 9.24175 | 60.6 % | 32 | 26.82 | 3.32131 | 7.6 % |
| 5 | 11.78 | 7.50875 | 33.8 % | 33 | 27.19 | 3.27759 | 7.4 % |
| 6 | 12.55 | 7.05041 | 1.0 % | 34 | 27.77 | 3.21017 | 11.8 % |
| 7 | 12.91 | 6.85401 | 15.7 % | 35 | 27.98 | 3.1863 | 10.3 % |
| 8 | 13.35 | 6.62927 | 34.6 % | 36 | 28.34 | 3.14713 | 9.7 % |
| 9 | 14.00 | 6.31959 | 16.1 % | 37 | 28.99 | 3.07733 | 0.5 % |
| 10 | 14.63 | 6.04886 | 5.9 % | 38 | 29.57 | 3.01822 | 2.5 % |
| 11 | 15.13 | 5.85254 | 0.3 % | 39 | 30.25 | 2.95205 | 1.7 % |
| 12 | 15.54 | 5.69788 | 0.4 % | 40 | 30.58 | 2.92079 | 7.1 % |
| 13 | 16.17 | 5.47711 | 11.1 % | 41 | 31.12 | 2.87158 | 20.7 % |
| 14 | 16.78 | 5.27789 | 100.0 % | 42 | 31.74 | 2.81698 | 0.3 % |
| 15 | 17.19 | 5.15429 | 57.9 % | 43 | 32.51 | 2.75216 | 3.0% |
| 16 | 18.03 | 4.91506 | 2.9 % | 44 | 33.08 | 2.70612 | 8.9 % |
| 17 | 18.54 | 4.7811 | 0.9 % | 45 | 33.87 | 2.64418 | 0.9 % |
| 18 | 19.21 | 4.61728 | 33.7 % | 46 | 34.26 | 2.6155 | 8.4 % |
| 19 | 20.14 | 4.40652 | 22.5 % | 47 | 34.95 | 2.56515 | 1.4 % |
| 20 | 20.54 | 4.32096 | 0.8 % | 48 | 35.40 | 2.53363 | 0.6 % |
| 21 | 21.59 | 4.11197 | 0.2 % | 49 | 35.84 | 2.50368 | 0.6 % |
| 22 | 22.22 | 3.99834 | 77.8 % | 50 | 36.80 | 2.4407 | 0.3 % |
| 23 | 22.48 | 3.95225 | 5.6 % | 51 | 37.17 | 2.41675 | 0.5 % |
| 24 | 22.99 | 3.8658 | 0.6 % | 52 | 37.61 | 2.38959 | 3.9 % |
| 25 | 23.39 | 3.8006 | 7.2 % | 53 | 37.69 | 2.38451 | 2.9 % |
| 26 | 23.85 | 3.72784 | 8.1 % | 54 | 39.00 | 2.30791 | 0.5 % |
| 27 | 24.14 | 3.68365 | 6.0 % | 55 | 39.25 | 2.2935 | 1.0 % |
| 28 | 24.87 | 3.57674 | 4.7 % | 56 | 39.70 | 2.26836 | 1.9 % |

The X-ray diffraction data of the crystal form B of the compound represented by formula (I) are shown in Table 4, and the corresponding pattern is shown in FIG. 8. The pattern was obtained using Cu-Kα radiation, and it had characteristic absorption peaks at diffraction 2θ angles of 6.65°, 8.39°, 9.56°, 11.78°, 12.91°, 13.35°, 14.00°, 14.63°, 16.17°, 16.78°, 17.19°, 19.21°, 20.14°, 22.22°, 23.39°, 23.85°, 24.14°, 25.15°, 26.13°, 26.82°, 27.19°, 27.77°, 27.98°, 28.34°, 30.58°, 31.12°, 33.08°, and 34.26°, wherein the error range of the 2θ angle for each characteristic peak was ±0.2°. The DSC profile showed a sharp endothermic peak at 214.71 °C, with an absorption peak onset at 213.77 °C and an enthalpy value of 88.316 J/g, as shown in FIG. 9. The TGA profile showed substantially no weight loss below 100 °C, a weight loss of about 0.517% (remaining weight: 99.483%) at 150.72 °C, and gradual melting and decomposition above 210 °C, as shown in FIG. 10.

### Example 5

Samples of the crystal form A of the compound represented by formula (I), the crystal form B of the compound represented by formula (I), the crystal form I of the p-toluenesulfonate of the compound represented by formula (I), and the crystal form I of the hydrochloride of the compound represented by formula (I) were separately spread out and left to stand in open containers, and subjected to 10-day and 30-day stability tests under light exposure (5000 Lux ± 500 Lux), high temperature (60 °C), and high humidity (25 °C, RH 90% ± 5%) conditions to investigate the stability of the crystal forms. The specific study data are shown in Table 5.

**Table 5. Comparison of stability data of crystal forms after standing for 30 days under different conditions**

| Sample name | Light exposure | 60 °C | High humidity |
|---|---|---|---|
| Crystal form A of compound represented by formula (I) | Crystal form A | Crystal form A | Mixed crystal |
| Crystal form B of compound represented by formula (I) | Crystal form B | Crystal form B | Crystal form B |
| Crystal form I of hydrochloride of compound represented by formula (I) | Crystal form I of hydrochloride | Crystal form I of hydrochloride | Crystal form I of hydrochloride |
| Crystal form I of p-toluenesulfonate of compound represented by formula (I) | Crystal form I of p-toluenesulfonate | Crystal form I of p-toluenesulfonate | Crystal form I of p-toluenesulfonate |

The data of the crystal form stability study indicated that, except for the crystal form A of the compound represented by formula (I), which had poor stability under high humidity conditions, the other crystal form samples exhibited good crystal form stability under light exposure, high temperature, and high humidity conditions.

### Example 6

Samples of the crystal form A of the compound represented by formula (I), the crystal form B of the compound represented by formula (I), the crystal form I of the p-toluenesulfonate of the compound represented by formula (I), and the crystal form I of the hydrochloride of the compound represented by formula (I) were separately spread out and left to stand in open containers, and subjected to 10-day and 30-day stability tests under light exposure (5000 Lux ± 500 Lux), high temperature (60 °C), and high humidity (25 °C, RH 90% ± 5%) conditions to investigate the stability of related substances. The specific study data are shown in Table 6.

**Table 6. Comparison of stability data of related substances under different conditions**

| Sample name | Time (days) | Light exposure | 60 °C | High humidity |
|---|---|---|---|---|
| Crystal form A of compound represented by formula (I) | 0 | 99.85% | 99.85% | 99.85% |
| | 10 | 99.11% | 99.82% | 99.83% |
| | 30 | 98.16% | 99.70% | 99.84% |
| Crystal form B of compound represented by formula (I) | 0 | 99.85% | 99.85% | 99.85% |
| | 10 | 98.54% | 99.85% | 99.85% |
| | 30 | 95.66% | 99.78% | 99.85% |
| Crystal form I of p-toluenesulfonate of compound represented by formula (I) | 0 | 99.44% | 99.44% | 99.44% |
| | 10 | 99.08% | 99.44% | 99.44% |
| | 30 | 98.95% | 99.42% | 99.44% |
| Crystal form I of hydrochloride of compound represented by formula (I) | 0 | 99.84% | 99.84% | 99.84% |
| | 10 | 99.60% | 99.51% | 98.84% |
| | 30 | 98.61% | 99.32% | 98.80% |

The results of the related substance stability investigation indicated that after salt formation, the chemical stability was improved to varying degrees. Under light exposure conditions, the four samples all exhibited degradation to varying degrees, suggesting that the samples should be stored protected from light. The stability of the crystal form I of the p-toluenesulfonate of the compound represented by formula (I) under high temperature conditions was superior to that of the crystal form I of the hydrochloride.

### Example 7

Samples of the prepared crystal form A of the compound represented by formula (I), the prepared crystal form B of the compound represented by formula (I), the crystal form I of the p-toluenesulfonate of the compound represented by formula (I) obtained according to Example 1, and the crystal form I of the hydrochloride of the compound represented by formula (I) obtained according to Example 2 were subjected to saturation solubility tests in water and at different pH values. The results showed that after salt formation, the solubility of the free base in water and at pH 1.0 was greatly increased. In the selected dissolution medium at pH 1.0, the p-toluenesulfonate of the compound represented by formula (I) showed the most significant increase in solubility. The specific study data are shown in Table 7.

**Table 7. Solubility data (mg/mL) under different conditions**

| Sample description | Crystal form A of compound represented by formula (I) | Crystal form B of compound represented by formula (I) | Crystal form I of hydrochloride of compound represented by formula (I) | Crystal form I of p-toluenesulfonate of compound represented by formula (I) |
|---|---|---|---|---|
| pH 1.0 | 0.29 mg/mL | 0.29 mg/mL | 0.29 mg/mL | 0.88 mg/mL |
| pH 4.5 | 1.32 mg/mL | 1.85 mg/mL | 2.43 mg/mL | 0.50 mg/mL |
| Water | 0.02 mg/mL | 0.01 mg/mL | 1.88 mg/mL | 0.44 mg/mL |
| pH 6.8 | 0.01 mg/mL | 0.02 mg/mL | 0.02 mg/mL | 0.02 mg/mL |

### Example 8

The crystal form I of the p-toluenesulfonate of the compound represented by formula (I) prepared according to the method of Example 1 was subjected to grinding, heating, and tableting treatments. The study results showed that the crystal form was stable. The detailed experimental data are shown in Table 8 below.

**Table 8. Stability study of crystal form I of p-toluenesulfonate**

| Sample | Treatment method | Experimental process | Crystal form | DSC peak value |
|---|---|---|---|---|
| Crystal form I of p-toluenesulfonate | Grinding for 10 min | 1 g of a sample of the crystal form I of the p-toluenesulfonate was taken and ground in a mortar for 10 min | Crystal form I of p-toluenesulfonate | 269.39 °C |
| Crystal form I of p-toluenesulfonate | Heating at 80 °C for 3 h | 1 g of a sample of the crystal form I of the p-toluenesulfonate was taken, spread out, and heated at 80 °C for 3 h | Crystal form I of p-toluenesulfonate | 269.38 °C |
| Crystal form I of p-toluenesulfonate | Tableting | A sample of the crystal form I of the p-toluenesulfonate was taken and compressed into tablets | Crystal form I of p-toluenesulfonate | 269.39 °C |

The results indicated that the crystal form I of the p-toluenesulfonate of the compound of formula (I) obtained in the present application had good stability.

### Example 9

Following the procedures in Example 1 of the present application, the compound represented by formula (I) was mixed with the acids and solvents shown in the table below, yet no crystal forms of salts were obtained.

| Acid | Solvent | Acid/base (mol/mol) | Results |
|---|---|---|---|
| Sulfuric acid | Ethanol | 0.5 | The crystal form A of the compound represented by formula (I) was obtained, and no salt was formed |
| | Acetone | 1.1 | The obtained crystal form product exhibited high hygroscopicity |
| Phosphoric acid | Ethanol | 1.1 | The obtained crystal form product exhibited high hygroscopicity |
| Citric acid | Ethanol | 2.1 | The obtained crystal form product was poor in crystalline state. |
| Acetic acid | Acetone | 1.1 | The crystal form A of the compound represented by formula (I) was obtained, and no salt was formed |
| L-Lactic acid | Acetone | 1.1 | The crystal form A of the compound represented by formula (I) was obtained, and no salt was formed |

The results showed that sulfuric acid, acetic acid, and L-lactic acid at a molar ratio of 0.5 failed to achieve salt formation; the crystal forms of other salts obtained exhibited problems such as high hygroscopicity, poor product properties, and poor crystalline purity.

### Example 10: Pharmacokinetic Experiment

### 10.1. Experimental animals

Number of animals: 6 Beagle dogs per group, half male and half female.

Grouping method: Dogs were randomly grouped by sex based on body weight.

### 10.2. Reagent preparation

A sample of the crystal form B of the compound represented by formula (I) was taken and added to a mixed solution of Solutol HS 15 and 20% HP-β-CD, and the resulting mixture was well mixed by ultrasonication and vortexing to give the desired reagent. Post-preparation storage condition and shelf life: the prepared reagent could be temporarily stored in a refrigerator at 2-8 °C for 3 h.

A sample of the crystal form I of the p-toluenesulfonate of the compound represented by formula (I) was taken and added to a mixed solution of Solutol HS 15 and 20% HP-β-CD, and the resulting mixture was well mixed by ultrasonication and vortexing to give the desired reagent.

A sample of the crystal form I of the p-toluenesulfonate of the compound represented by formula (I) was taken and added to a mixed solution of SBE-β-CD and 50 mM sodium acetate (pH 4.61), and the resulting mixture was well mixed by ultrasonication and vortexing to give the desired reagent. Post-preparation storage condition and shelf life: the prepared reagent could be temporarily stored in a refrigerator at 2-8 °C for 3 h.

### 10.3. Experimental procedures

Administration dose: 10 mg/kg, calculated based on the content of the compound represented by formula (I).

Administration concentration: 2 mg/mL.

Administration volume: 5 mL/kg.

Administration frequency: single administration.

Administration route: oral gavage.

Sampling time points: before administration, and 15 min (± 1 min), 30 min (± 1 min), 1 h (± 2 min), 2 h (± 2 min), 4 h (± 5 min), 8 h (± 5 min), and 24 h (± 20 min) after administration.

Sampling animals: all surviving dogs in each group.

Sampling site: forelimb vein or other suitable veins.

Sampling amount: about 0.5 mL, with heparin sodium as an anticoagulant.

Blood sample treatment: The collected blood samples were each added into a centrifuge tube containing the anticoagulant and mixed well with the anticoagulant by gentle shaking. The whole blood samples were placed in an ice box protected from light before centrifugation. They were then centrifuged at 1800 rpm for 10 min at 2-8 °C within 2 h to separate plasma. The plasma was divided into 2 aliquots and stored at -66 °C or below until analysis.

Data processing and analysis: Pharmacokinetic parameters including Cₘₐₓ, Tₘₐₓ, AUCₗₐₛₜ, and AUC_{0-∞} were calculated using a non-compartmental model.

Tₘₐₓ and Cₘₐₓ were obtained from measured values.

AUC₀₋ₜ was calculated using the linear trapezoidal method, where t represents the time of the last quantifiable plasma concentration;
${AUC}_{0 - ∞} = {AUC}_{0 - t} + {C}_{t} / {λ}_{z} ,$ where Cₜ represents the plasma concentration at the last time point with a quantifiable concentration, and λ_{z} represents the terminal elimination rate constant.

### 10.4. Experimental results

The results are shown in Table 9 below.

**Table 9. Pharmacokinetic data**

| Test sample | Vehicle | AUCₗₐₛₜ | AUC_{0-∞} | Cₘₐₓ | Tₘₐₓ |
|---|---|---|---|---|---|
| | | (h*ng/mL) | (h*ng/mL) | (ng/mL) | (h) |
| Crystal form B of compound represented by formula (I) | Solutol HS 15 and 20% HP-β-CD | 1640±730 | 1390±356 | 378±359 | 1.3±1.3 |
| Crystal form I of p-toluenesulfonate of compound represented by formula (I) | Solutol HS 15 and 20% HP-β-CD | 2190±689 | 2220±691 | 448±123 | 0.50±0.0 |
| | SBE-β-CD and 50 mM sodium acetate | 2680±1150 | 2720±1170 | 1080±636 | 0.54±0.25 |

The results showed that in the oral gavage experiment in Beagle dogs, compared with the free base, the p-toluenesulfonate of the compound of formula (I) exhibited higher AUC and Cₘₐₓ, shorter time to peak *in vivo*, and greater exposure, indicating that the p-toluenesulfonate of the compound of formula (I) possessed favorable pharmacokinetic properties.

Although specific embodiments of the present disclosure are described above, it will be appreciated by those skilled in the art that these are only illustrative and that many alterations or modifications can be made to these embodiments without departing from the principles and spirit of the present disclosure. Therefore, the scope of protection of the present disclosure is defined by the appended claims.

## Claims

1. A p-toluenesulfonate or hydrochloride of a compound represented by formula (I), 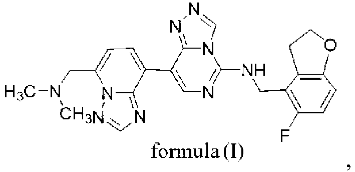
wherein the molar ratio of the compound represented by formula (I) to p-toluenesulfonic acid is 1:1;
the molar ratio of the compound represented by formula (I) to hydrochloric acid is 1:1.

2. A crystal form of a compound represented by formula (I) or a salt thereof, being a crystal form I of a p-toluenesulfonate of the compound represented by formula (I), a crystal form I of a hydrochloride of the compound represented by formula (I), or a crystal form B of the compound represented by formula (I),
wherein the structure of the compound represented by formula (I) is: 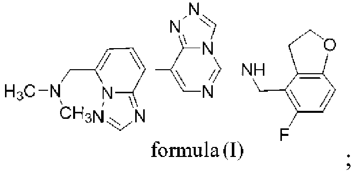
an X-ray powder diffraction pattern of the crystal form I of the p-toluenesulfonate of the compound represented by formula (I), measured using Cu-Kα radiation and expressed in terms of 2θ angles, has diffraction peaks at 6.63±0.20°, 11.91±0.20°, 13.24±0.20°, 14.03±0.20°, 15.71±0.20°, and 18.14±0.20°;
in the crystal form I of the p-toluenesulfonate of the compound represented by formula (I), the molar ratio of the compound represented by formula (I) to p-toluenesulfonic acid is 1:1;
an X-ray powder diffraction pattern of the crystal form I of the hydrochloride of the compound represented by formula (I), measured using Cu-Kα radiation and expressed in terms of 2θ angles, has diffraction peaks at 7.89±0.20°, 11.61±0.20°, 15.89±0.20°, 25.58±0.20°, and 26.45±0.20°; in the crystal form I of the hydrochloride of the compound represented by formula (I), the molar ratio of the compound represented by formula (I) to hydrochloric acid is 1:1;
an X-ray powder diffraction pattern of the crystal form B of the compound represented by formula (I), measured using Cu-Kα radiation and expressed in terms of 2θ angles, has diffraction peaks at 8.39±0.20°, 9.56±0.20°, 16.78±0.20°, 17.19±0.20°, 22.22±0.20°, and 25.15±0.20°.

3. The crystal form of the compound represented by formula (I) or the salt thereof according to claim 2, wherein the crystal form of the compound represented by formula (I) or the salt thereof meets one or more of the following conditions:
(1) the X-ray powder diffraction pattern of the crystal form I of the p-toluenesulfonate of the compound represented by formula (I), measured using Cu-Kα radiation and expressed in terms of 2θ angles, further has diffraction peaks at one or more of 17.24±0.20°, 21.92±0.20°, 22.54±0.20°, 24.79±0.20°, 25.06±0.20°, and 26.63±0.20°;
(2) the X-ray powder diffraction pattern of the crystal form I of the hydrochloride of the compound represented by formula (I), measured using Cu-Kα radiation and expressed in terms of 2θ angles, further has diffraction peaks at one or more of 6.87±0.20°, 16.94±0.20°, 18.72±0.20°, 23.34±0.20°, and 23.96±0.20°;
(3) the X-ray powder diffraction pattern of the crystal form B of the compound represented by formula (I), measured using Cu-Kα radiation and expressed in terms of 2θ angles, further has diffraction peaks at one or more of 11.78±0.20°, 13.35±0.20°, 19.21±0.20°, 20.14±0.20°, 26.13±0.20°, and 31.12±0.20°;
preferably, the crystal form of the compound represented by formula (I) or the salt thereof meets one or more of the following conditions:
(1) the X-ray powder diffraction pattern of the crystal form I of the p-toluenesulfonate of the compound represented by formula (I), measured using Cu-Kα radiation and expressed in terms of 2θ angles, further has diffraction peaks at one or more of 10.87±0.20°, 14.29±0.20°, 17.70±0.20°, 18.99±0.20°, 21.63±0.20°, and 33.21±0.20°;
(2) the X-ray powder diffraction pattern of the crystal form I of the hydrochloride of the compound represented by formula (I), measured using Cu-Kα radiation and expressed in terms of 2θ angles, further has diffraction peaks at one or more of 15.57±0.20°, 28.97±0.20°, 31.46±0.20°, and 39.23±0.20°;
(3) the X-ray powder diffraction pattern of the crystal form B of the compound represented by formula (I), measured using Cu-Kα radiation and expressed in terms of 2θ angles, further has diffraction peaks at one or more of 6.65±0.20°, 12.91±0.20°, 14.00±0.20°, 16.17±0.20°, 27.77±0.20°, and 27.98±0.20°.

4. The crystal form of the compound represented by formula (I) or the salt thereof according to claim 2, wherein the crystal form of the compound represented by formula (I) or the salt thereof meets one or more of the following conditions:
(1) the X-ray powder diffraction pattern of the crystal form I of the p-toluenesulfonate of the compound represented by formula (I), measured using Cu-Kα radiation and expressed in terms of 2θ angles, has diffraction peaks at 6.63±0.20°, 10.87±0.20°, 11.91±0.20°, 13.24±0.20°, 14.03±0.20°, 14.29±0.20°, 15.71±0.20°, 17.24±0.20°, 18.14±0.20°, 22.54±0.20°, 24.79±0.20°, 25.06±0.20°, and 26.63±0.20°;
(2) the X-ray powder diffraction pattern of the crystal form I of the hydrochloride of the compound represented by formula (I), measured using Cu-Kα radiation and expressed in terms of 2θ angles, has diffraction peaks at 6.87±0.20°, 7.89±0.20°, 9.25±0.20°, 11.61±0.20°, 12.52±0.20°, 12.98±0.20°, 14.34±0.20°, 15.57±0.20°, 15.89±0.20°, 16.94±0.20°, 17.44±0.20°, 18.72±0.20°, 19.23±0.20°, 20.55±0.20°, 22.22±0.20°, 23.34±0.20°, 23.96±0.20°, 24.46±0.20°, 24.81±0.20°, 25.27±0.20°, 25.58±0.20°, 26.45±0.20°, 27.48±0.20°, 27.92±0.20°, 28.97±0.20°, and 31.46±0.20°;
(3) the X-ray powder diffraction pattern of the crystal form B of the compound represented by formula (I), measured using Cu-Kα radiation and expressed in terms of 2θ angles, has diffraction peaks at 6.65±0.20°, 8.39±0.20°, 9.56±0.20°, 11.78±0.20°, 12.91±0.20°, 13.35±0.20°, 14.00±0.20°, 14.63±0.20°, 16.17±0.20°, 16.78±0.20°, 17.19±0.20°, 19.21±0.20°, 20.14±0.20°, 22.22±0.20°, 23.39±0.20°, 23.85±0.20°, 24.14±0.20°, 25.15±0.20°, 26.13±0.20°, 26.82±0.20°, 27.19±0.20°, 27.77±0.20°, 27.98±0.20°, 28.34±0.20°, 30.58±0.20°, 31.12±0.20°, 33.08±0.20°, and 34.26±0.20°.

5. The crystal form of the compound represented by formula (I) or the salt thereof according to claim 2, wherein the crystal form of the compound represented by formula (I) or the salt thereof meets one or more of the following conditions:
(1) the X-ray powder diffraction pattern of the crystal form I of the p-toluenesulfonate of the compound represented by formula (I), measured using Cu-Kα radiation and expressed in terms of 2θ angles, exhibits diffraction peak positions as shown in the following table:
| No. | 2θ (°) | No. | 2θ (°) | No. | 2θ (°) | No. | 2θ (°) |
|---|---|---|---|---|---|---|---|
| 1 | 5.98 | 23 | 23.26 | 45 | 34.56 | 67 | 47.19 |
| 2 | 6.63 | 24 | 23.55 | 46 | 34.91 | 68 | 47.45 |
| 3 | 8.82 | 25 | 24.06 | 47 | 35.11 | 69 | 48.19 |
| 4 | 10.87 | 26 | 24.79 | 48 | 35.68 | 70 | 49.04 |
| 5 | 11.91 | 27 | 25.06 | 49 | 36.18 | 71 | 49.64 |
| 6 | 13.24 | 28 | 26.03 | 50 | 36.75 | 72 | 50.02 |
| 7 | 14.03 | 29 | 26.63 | 51 | 36.99 | 73 | 50.56 |
| 8 | 14.29 | 30 | 27.27 | 52 | 37.49 | 74 | 50.71 |
| 9 | 15.71 | 31 | 27.49 | 53 | 37.95 | 75 | 51.59 |
| 10 | 16.37 | 32 | 28.02 | 54 | 38.28 | 76 | 52.00 |
| 11 | 17.24 | 33 | 28.32 | 55 | 38.67 | 77 | 52.39 |
| 12 | 17.70 | 34 | 28.68 | 56 | 39.19 | 78 | 53.51 |
| 13 | 18.14 | 35 | 29.23 | 57 | 40.40 | 79 | 54.70 |
| 14 | 18.99 | 36 | 29.84 | 58 | 40.91 | 80 | 55.04 |
| 15 | 19.76 | 37 | 30.20 | 59 | 41.57 | 81 | 55.81 |
| 16 | 20.14 | 38 | 30.71 | 60 | 41.99 | 82 | 56.39 |
| 17 | 20.44 | 39 | 31.13 | 61 | 42.79 | 83 | 56.96 |
| 18 | 20.86 | 40 | 31.59 | 62 | 43.29 | 84 | 57.76 |
| 19 | 21.24 | 41 | 32.30 | 63 | 43.98 | 85 | 58.41 |
| 20 | 21.63 | 42 | 32.79 | 64 | 44.82 | 86 | 58.91 |
| 21 | 21.92 | 43 | 33.21 | 65 | 45.82 | 87 | 59.45 |
| 22 | 22.54 | 44 | 33.90 | 66 | 46.66 | | |
(2) the X-ray powder diffraction pattern of the crystal form I of the hydrochloride of the compound represented by formula (I), measured using Cu-Kα radiation and expressed in terms of 2θ angles, exhibits diffraction peak positions as shown in the following table:
| No. | 2θ (°) | No. | 2θ (°) |
|---|---|---|---|
| 1 | 6.87 | 24 | 25.58 |
| 2 | 7.89 | 25 | 26.45 |
| 3 | 9.25 | 26 | 27.48 |
| 4 | 11.61 | 27 | 27.92 |
| 5 | 12.52 | 28 | 28.11 |
| 6 | 12.98 | 29 | 28.97 |
| 7 | 13.27 | 30 | 29.34 |
| 8 | 14.34 | 31 | 30.34 |
| 9 | 15.57 | 32 | 30.84 |
| 10 | 15.89 | 33 | 31.46 |
| 11 | 16.94 | 34 | 32.16 |
| 12 | 17.44 | 35 | 32.61 |
| 13 | 17.74 | 36 | 33.10 |
| 14 | 18.72 | 37 | 33.75 |
| 15 | 19.23 | 38 | 34.33 |
| 16 | 20.27 | 39 | 34.63 |
| 17 | 20.55 | 40 | 35.46 |
| 18 | 22.22 | 41 | 35.88 |
| 19 | 23.34 | 42 | 36.76 |
| 20 | 23.96 | 43 | 37.57 |
| 21 | 24.46 | 44 | 38.03 |
| 22 | 24.81 | 45 | 39.23 |
| 23 | 25.27 | | |
;
(3) the X-ray powder diffraction pattern of the crystal form B of the compound represented by formula (I), measured using Cu-Kα radiation and expressed in terms of 2θ angles, exhibits diffraction peak positions as shown in the following table:
| No. | 2θ (°) | No. | 2θ (°) |
|---|---|---|---|
| 1 | 6.65 | 29 | 25.15 |
| 2 | 7.95 | 30 | 25.65 |
| 3 | 8.39 | 31 | 26.13 |
| 4 | 9.56 | 32 | 26.82 |
| 5 | 11.78 | 33 | 27.19 |
| 6 | 12.55 | 34 | 27.77 |
| 7 | 12.91 | 35 | 27.98 |
| 8 | 13.35 | 36 | 28.34 |
| 9 | 14.00 | 37 | 28.99 |
| 10 | 14.63 | 38 | 29.57 |
| 11 | 15.13 | 39 | 30.25 |
| 12 | 15.54 | 40 | 30.58 |
| 13 | 16.17 | 41 | 31.12 |
| 14 | 16.78 | 42 | 31.74 |
| 15 | 17.19 | 43 | 32.51 |
| 16 | 18.03 | 44 | 33.08 |
| 17 | 18.54 | 45 | 33.87 |
| 18 | 19.21 | 46 | 34.26 |
| 19 | 20.14 | 47 | 34.95 |
| 20 | 20.54 | 48 | 35.40 |
| 21 | 21.59 | 49 | 35.84 |
| 22 | 22.22 | 50 | 36.80 |
| 23 | 22.48 | 51 | 37.17 |
| 24 | 22.99 | 52 | 37.61 |
| 25 | 23.39 | 53 | 37.69 |
| 26 | 23.85 | 54 | 39.00 |
| 27 | 24.14 | 55 | 39.25 |
| 28 | 24.87 | 56 | 39.70 |
;
preferably, the crystal form of the compound represented by formula (I) or the salt thereof meets one or more of the following conditions:
(1) the X-ray powder diffraction pattern of the crystal form I of the p-toluenesulfonate of the compound represented by formula (I), measured using Cu-Kα radiation and expressed in terms of 2θ angles, exhibits diffraction peak positions and relative intensities as shown in the following table:
| No. | 2θ (°) | Relative intensity (%) | No. | 2θ (°) | Relative intensity (%) |
|---|---|---|---|---|---|
| 1 | 5.98 | 0.3 | 45 | 34.56 | 0.5 |
| 2 | 6.63 | 93.0 | 46 | 34.91 | 0.3 |
| 3 | 8.82 | 0.2 | 47 | 35.11 | 0.2 |
| 4 | 10.87 | 3.7 | 48 | 35.68 | 0.5 |
| 5 | 11.91 | 26.2 | 49 | 36.18 | 0.6 |
| 6 | 13.24 | 100.0 | 50 | 36.75 | 0.3 |
| 7 | 14.03 | 11.5 | 51 | 36.99 | 0.3 |
| 8 | 14.29 | 3.6 | 52 | 37.49 | 0.4 |
| 9 | 15.71 | 12.9 | 53 | 37.95 | 0.1 |
| 10 | 16.37 | 0.2 | 54 | 38.28 | 0.1 |
| 11 | 17.24 | 6.7 | 55 | 38.67 | 0.5 |
| 12 | 17.70 | 2.5 | 56 | 39.19 | 0.1 |
| 13 | 18.14 | 12.8 | 57 | 40.40 | 2.1 |
| 14 | 18.99 | 2.1 | 58 | 40.91 | 0.5 |
| 15 | 19.76 | 0.1 | 59 | 41.57 | 0.3 |
| 16 | 20.14 | 0.2 | 60 | 41.99 | 1.5 |
| 17 | 20.44 | 0.2 | 61 | 42.79 | 0.1 |
| 18 | 20.86 | 1.0 | 62 | 43.29 | 0.3 |
| 19 | 21.24 | 1.9 | 63 | 43.98 | 0.5 |
| 20 | 21.63 | 3.2 | 64 | 44.82 | 0.6 |
| 21 | 21.92 | 5.8 | 65 | 45.82 | 0.1 |
| 22 | 22.54 | 5.4 | 66 | 46.66 | 0.0 |
| 23 | 23.26 | 0.3 | 67 | 47.19 | 1.6 |
| 24 | 23.55 | 0.7 | 68 | 47.45 | 0.3 |
| 25 | 24.06 | 1.4 | 69 | 48.19 | 0.4 |
| 26 | 24.79 | 5.3 | 70 | 49.04 | 0.6 |
| 27 | 25.06 | 6.3 | 71 | 49.64 | 0.2 |
| 28 | 26.03 | 1.8 | 72 | 50.02 | 0.3 |
| 29 | 26.63 | 8.8 | 73 | 50.56 | 0.2 |
| 30 | 27.27 | 0.5 | 74 | 50.71 | 0.2 |
| 31 | 27.49 | 0.9 | 75 | 51.59 | 0.2 |
| 32 | 28.02 | 1.1 | 76 | 52.00 | 0.1 |
| 33 | 28.32 | 0.9 | 77 | 52.39 | 0.1 |
| 34 | 28.68 | 0.3 | 78 | 53.51 | 0.1 |
| 35 | 29.23 | 0.1 | 79 | 54.70 | 0.2 |
| 36 | 29.84 | 0.3 | 80 | 55.04 | 0.4 |
| 37 | 30.20 | 0.4 | 81 | 55.81 | 0.1 |
| 38 | 30.71 | 1.1 | 82 | 56.39 | 0.1 |
| 39 | 31.13 | 0.3 | 83 | 56.96 | 0.1 |
| 40 | 31.59 | 1.0 | 84 | 57.76 | 0.2 |
| 41 | 32.30 | 1.1 | 85 | 58.41 | 0.1 |
| 42 | 32.79 | 0.4 | 86 | 58.91 | 0.0 |
| 43 | 33.21 | 3.2 | 87 | 59.45 | 0.1 |
| 44 | 33.90 | 0.8 | | | |
(2) the X-ray powder diffraction pattern of the crystal form I of the hydrochloride of the compound represented by formula (I), measured using Cu-Kα radiation and expressed in terms of 2θ angles, exhibits diffraction peak positions and relative intensities as shown in the following table:
| No. | 2θ (°) | Relative intensity (%) | No. | 2θ (°) | Relative intensity (%) |
|---|---|---|---|---|---|
| 1 | 6.87 | 3.8 | 24 | 25.58 | 8.4 |
| 2 | 7.89 | 100.0 | 25 | 26.45 | 30.4 |
| 3 | 9.25 | 0.5 | 26 | 27.48 | 0.3 |
| 4 | 11.61 | 12.7 | 27 | 27.92 | 0.8 |
| 5 | 12.52 | 1.7 | 28 | 28.11 | 0.9 |
| 6 | 12.98 | 0.4 | 29 | 28.97 | 2.2 |
| 7 | 13.27 | 0.1 | 30 | 29.34 | 1.0 |
| 8 | 14.34 | 0.9 | 31 | 30.34 | 0.6 |
| 9 | 15.57 | 2.3 | 32 | 30.84 | 0.4 |
| 10 | 15.89 | 51.2 | 33 | 31.46 | 2.1 |
| 11 | 16.94 | 2.6 | 34 | 32.16 | 0.4 |
| 12 | 17.44 | 1.2 | 35 | 32.61 | 0.2 |
| 13 | 17.74 | 0.2 | 36 | 33.10 | 0.3 |
| 14 | 18.72 | 4.2 | 37 | 33.75 | 0.3 |
| 15 | 19.23 | 0.5 | 38 | 34.33 | 0.2 |
| 16 | 20.27 | 0.3 | 39 | 34.63 | 0.2 |
| 17 | 20.55 | 1.7 | 40 | 35.46 | 0.4 |
| 18 | 22.22 | 1.7 | 41 | 35.88 | 0.1 |
| 19 | 23.34 | 6.2 | 42 | 36.76 | 0.3 |
| 20 | 23.96 | 4.3 | 43 | 37.57 | 0.8 |
| 21 | 24.46 | 1.1 | 44 | 38.03 | 1.7 |
| 22 | 24.81 | 1.5 | 45 | 39.23 | 2.0 |
| 23 | 25.27 | 0.6 | | | |
(3) the X-ray powder diffraction pattern of the crystal form B of the compound represented by formula (I), measured using Cu-Kα radiation and expressed in terms of 2θ angles, exhibits diffraction peak positions and relative intensities as shown in the following table:
| No. | 2θ (°) | Relative intensity | No. | 2θ (°) | Relative intensity |
|---|---|---|---|---|---|
| 1 | 6.65 | 18.0 % | 29 | 25.15 | 66.1 % |
| 2 | 7.95 | 1.4 % | 30 | 25.65 | 6.5 % |
| 3 | 8.39 | 95.1 % | 31 | 26.13 | 35.5 % |
| 4 | 9.56 | 60.6 % | 32 | 26.82 | 7.6 % |
| 5 | 11.78 | 33.8 % | 33 | 27.19 | 7.4 % |
| 6 | 12.55 | 1.0 % | 34 | 27.77 | 11.8 % |
| 7 | 12.91 | 15.7 % | 35 | 27.98 | 10.3 % |
| 8 | 13.35 | 34.6 % | 36 | 28.34 | 9.7 % |
| 9 | 14.00 | 16.1 % | 37 | 28.99 | 0.5 % |
| 10 | 14.63 | 5.9 % | 38 | 29.57 | 2.5 % |
| 11 | 15.13 | 0.3 % | 39 | 30.25 | 1.7 % |
| 12 | 15.54 | 0.4 % | 40 | 30.58 | 7.1 % |
| 13 | 16.17 | 11.1 % | 41 | 31.12 | 20.7 % |
| 14 | 16.78 | 100.0 % | 42 | 31.74 | 0.3 % |
| 15 | 17.19 | 57.9 % | 43 | 32.51 | 3.0 % |
| 16 | 18.03 | 2.9 % | 44 | 33.08 | 8.9 % |
| 17 | 18.54 | 0.9 % | 45 | 33.87 | 0.9 % |
| 18 | 19.21 | 33.7 % | 46 | 34.26 | 8.4 % |
| 19 | 20.14 | 22.5 % | 47 | 34.95 | 1.4 % |
| 20 | 20.54 | 0.8 % | 48 | 35.40 | 0.6 % |
| 21 | 21.59 | 0.2 % | 49 | 35.84 | 0.6 % |
| 22 | 22.22 | 77.8 % | 50 | 36.80 | 0.3 % |
| 23 | 22.48 | 5.6 % | 51 | 37.17 | 0.5 % |
| 24 | 22.99 | 0.6 % | 52 | 37.61 | 3.9 % |
| 25 | 23.39 | 7.2 % | 53 | 37.69 | 2.9 % |
| 26 | 23.85 | 8.1 % | 54 | 39.00 | 0.5 % |
| 27 | 24.14 | 6.0 % | 55 | 39.25 | 1.0 % |
| 28 | 24.87 | 4.7 % | 56 | 39.70 | 1.9 % |
more preferably, the crystal form of the compound represented by formula (I) or the salt thereof meets one or more of the following conditions:
(1) the X-ray powder diffraction pattern of the crystal form I of the p-toluenesulfonate of the compound represented by formula (I), measured using Cu-Kα radiation and expressed in terms of 2θ angles, is substantially as shown in FIG. 1;
(2) the X-ray powder diffraction pattern of the crystal form I of the hydrochloride of the compound represented by formula (I), measured using Cu-Kα radiation and expressed in terms of 2θ angles, is substantially as shown in FIG. 4;
(3) the X-ray powder diffraction pattern of the crystal form B of the compound represented by formula (I), measured using Cu-Kα radiation and expressed in terms of 2θ angles, is substantially as shown in FIG. 8.

6. The crystal form of the compound represented by formula (I) or the salt thereof according to claim 2, wherein the crystal form of the compound represented by formula (I) or the salt thereof meets one or more of the following conditions:
(1) a differential scanning calorimetry curve of the crystal form I of the p-toluenesulfonate of the compound represented by formula (I) has an endothermic peak onset at 266.75±3 °C, and/or the differential scanning calorimetry curve of the crystal form I of the p-toluenesulfonate of the compound represented by formula (I) has a peak temperature of 269.39±3 °C;
(2) a thermogravimetric analysis curve of the crystal form I of the p-toluenesulfonate of the compound represented by formula (I) shows no weight loss below 100±3 °C, for example, no weight loss in a range of 25±3 °C to 100±3 °C, such as no weight loss in a range of 50±3 °C to 100±3 °C;
(3) the thermogravimetric analysis curve of the crystal form I of the p-toluenesulfonate of the compound represented by formula (I) shows that the crystal form undergoes decomposition above 250±3 °C;
(4) a differential scanning calorimetry curve of the crystal form I of the hydrochloride of the compound represented by formula (I) shows no endothermic peak below 250±3 °C, for example, no endothermic peak in a range of 40±3 °C to 250±3 °C, such as no endothermic peak in a range of 80±3 °C to 250±3 °C;
(5) a thermogravimetric analysis curve of the crystal form I of the hydrochloride of the compound represented by formula (I) shows a weight loss of 0.625% at 101.04±3 °C, for example, a weight loss of 0.625% in a range of 25±3 °C to 101.04±3 °C;
(6) a differential scanning calorimetry curve of the crystal form B of the compound represented by formula (I) has an endothermic peak onset at 213.77±3 °C, and/or the differential scanning calorimetry curve of the crystal form B of the compound represented by formula (I) has a peak temperature of 214.71±3 °C;
(7) a thermogravimetric analysis curve of the crystal form B of the compound represented by formula (I) shows a weight loss of 0.517% at 150.72±3 °C, for example, a weight loss of 0.517% in a range of 25±3 °C to 150.72±3 °C;
(8) the thermogravimetric analysis curve of the crystal form B of the compound represented by formula (I) shows that the crystal form undergoes decomposition above 210±3 °C;
preferably, the crystal form of the compound represented by formula (I) or the salt thereof meets one or more of the following conditions:
(1) the differential scanning calorimetry curve of the crystal form I of the p-toluenesulfonate of the compound represented by formula (I) is substantially as shown in FIG. 2;
(2) the thermogravimetric analysis curve of the crystal form I of the p-toluenesulfonate of the compound represented by formula (I) is substantially as shown in FIG. 3;
(3) the differential scanning calorimetry curve of the crystal form I of the hydrochloride of the compound represented by formula (I) is substantially as shown in FIG. 5;
(4) the thermogravimetric analysis curve of the crystal form I of the hydrochloride of the compound represented by formula (I) is substantially as shown in FIG. 6;
(5) the differential scanning calorimetry curve of the crystal form B of the compound represented by formula (I) is substantially as shown in FIG. 9;
(6) the thermogravimetric analysis curve of the crystal form B of the compound represented by formula (I) is substantially as shown in FIG. 10.

7. A preparation method for the crystal form I of the p-toluenesulfonate of the compound represented by formula (I) according to any one of claims 2-6, the crystal form I of the hydrochloride of the compound represented by formula (I) according to any one of claims 2-6, or the crystal form B of the compound represented by formula (I) according to any one of claims 2-6, wherein
the preparation method for the crystal form I of the p-toluenesulfonate of the compound represented by formula (I) comprises the following step: crystallizing a mixture of p-toluenesulfonic acid, the compound represented by formula (I), and a solvent to obtain the crystal form I of the p-toluenesulfonate of the compound represented by formula (I), wherein the solvent is an alcohol solvent or a mixed solvent of an alcohol solvent and water;
the preparation method for the crystal form I of the hydrochloride of the compound represented by formula (I) comprises the following step: crystallizing a mixture of hydrochloric acid, the compound represented by formula (I), and a solvent to obtain the crystal form I of the hydrochloride of the compound represented by formula (I), wherein the solvent is an alcohol solvent or a mixed solvent of an alcohol solvent and water;
the preparation method for the crystal form B of the compound represented by formula (I) comprises the following step: crystallizing a mixture of the compound represented by formula (I) and a solvent to obtain the crystal form B of the compound represented by formula (I), wherein the solvent is an ester solvent.

8. The preparation method for the crystal form I of the p-toluenesulfonate of the compound represented by formula (I), the crystal form I of the hydrochloride of the compound represented by formula (I), or the crystal form B of the compound represented by formula (I) according to claim 7, wherein one or more of the following conditions are met:
(1) in the preparation method for the crystal form I of the p-toluenesulfonate of the compound represented by formula (I), the alcohol solvent is a C₁-C₃ alcohol solvent, for example, methanol, ethanol, n-propanol, or isopropanol, such as ethanol;
preferably, in the preparation method for the crystal form I of the p-toluenesulfonate of the compound represented by formula (I), the mixed solvent of the alcohol solvent and water is a mixed solvent of ethanol and water in a volume ratio of 3:1;
(2) in the preparation method for the crystal form I of the p-toluenesulfonate of the compound represented by formula (I), the p-toluenesulfonic acid is p-toluenesulfonic acid monohydrate;
(3) in the preparation method for the crystal form I of the p-toluenesulfonate of the compound represented by formula (I), the crystallization is cooling crystallization;
(4) the preparation method for the crystal form I of the p-toluenesulfonate of the compound represented by formula (I) further comprises filtering and drying, wherein the drying is preferably drying *in vacuo* under reduced pressure at 40-60 °C;
(5) in the preparation method for the crystal form I of the hydrochloride of the compound represented by formula (I), the alcohol solvent is a C₁-C₃ alcohol solvent, for example, methanol, ethanol, n-propanol, or isopropanol, such as ethanol;
preferably, in the preparation method for the crystal form I of the hydrochloride of the compound represented by formula (I), the mixed solvent of the alcohol solvent and water is a mixed solvent of ethanol and water in a volume ratio of 3:1;
(6) in the preparation method for the crystal form I of the hydrochloride of the compound represented by formula (I), the crystallization is cooling crystallization;
(7) the preparation method for the crystal form I of the hydrochloride of the compound represented by formula (I) further comprises filtering and drying, wherein the drying is preferably drying *in vacuo* under reduced pressure at 40-60 °C;
(8) in the preparation method for the crystal form B of the compound represented by formula (I), the ester solvent is ethyl acetate;
(9) in the preparation method for the crystal form B of the compound represented by formula (I), the crystallization is slurrying;
(10) in the preparation method for the crystal form B of the compound represented by formula (I), the compound represented by formula (I) is a crystal form A of the compound represented by formula (I).

9. A pharmaceutical composition, comprising a substance Z and one or more pharmaceutically acceptable auxiliary materials, wherein the substance Z is the p-toluenesulfonate of the compound represented by formula (I) according to claim 1, the hydrochloride of the compound represented by formula (I) according to claim 1, the crystal form I of the p-toluenesulfonate of the compound represented by formula (I) according to any one of claims 2-6, the crystal form I of the hydrochloride of the compound represented by formula (I) according to any one of claims 2-6, or the crystal form B of the compound represented by formula (I) according to any one of claims 2-6.

10. Use of a substance Z or the pharmaceutical composition according to claim 9 in the manufacture of a medicament for preventing and/or treating a disease associated with the mechanism of action of an EED protein and/or a PRC2 protein complex, wherein the substance Z is the p-toluenesulfonate of the compound represented by formula (I) according to claim 1, the hydrochloride of the compound represented by formula (I) according to claim 1, the crystal form I of the p-toluenesulfonate of the compound represented by formula (I) according to any one of claims 2-6, the crystal form I of the hydrochloride of the compound represented by formula (I) according to any one of claims 2-6, or the crystal form B of the compound represented by formula (I) according to any one of claims 2-6;
preferably, the disease is a tumor, for example, a malignant tumor, such as lymphoma, leukemia, multiple myeloma, mesothelioma, gastric cancer, malignant rhabdoid tumor, liver cancer, prostate cancer, breast cancer, brain tumor including neuroblastoma, glioma, glioblastoma and astrocytoma, cervical cancer, colon cancer, melanoma, endometrial cancer, esophageal cancer, head and neck cancer, lung cancer, nasopharyngeal cancer, ovarian cancer, pancreatic cancer, renal cancer, rectal cancer, thyroid cancer, parathyroid tumor, uterine tumor, and soft tissue tumor, wherein the lymphoma is preferably diffuse large B-cell lymphoma, follicular lymphoma, or non-Hodgkin's lymphoma.
